Europäisches Patentamt
European Patent Office
Office européen des brevets

(19)

(11) Veröffentlichungsnummer: **0 129 791**
**A2**

(12)

# EUROPÄISCHE PATENTANMELDUNG

(21) Anmeldenummer: 84106841.4

(22) Anmeldetag: 15.06.84

(51) Int. Cl.⁴: C 07 D 237/04
C 07 D 307/32, C 07 D 401/12
C 07 D 403/04, C 07 D 403/12
C 07 D 405/04, C 07 D 405/06
C 07 D 405/12, C 07 D 407/06
C 07 D 409/04, A 61 K 31/50
//C07D413/12

(30) Priorität: 20.06.83 DE 3322079
30.03.84 DE 3411850

(43) Veröffentlichungstag der Anmeldung:
02.01.85 Patentblatt 85/1

(84) Benannte Vertragsstaaten:
AT BE CH DE FR GB IT LI NL SE

(71) Anmelder: CASSELLA Aktiengesellschaft
Hanauer Landstrasse 526
D-6000 Frankfurt am Main 61(DE)

(72) Erfinder: Zoller, Gerhard, Dr.
Langwiese 16
D-6457 Maintal(DE)

(72) Erfinder: Beyerle, Rudi, Dr.
An der Pfaffenmauer 44
D-6000 Frankfurt/Main 60(DE)

(72) Erfinder: Just, Melitta, Dr.
Hüttenweg 6
D-6369 Schöneck(DE)

(72) Erfinder: Martorana, Piero, Dr.
Kaiser Friedrich Promenade 108
D-6380 Bad Homburg(DE)

(72) Erfinder: Bohn, Helmut, Dr.
Kranzbergring 11
D-6369 Schöneck(DE)

(72) Erfinder: Nitz, Rolf-Eberhard, Dr.
Heinrich-Bingemer-Weg 64
D-6000 Frankfurt/Main 60(DE)

(74) Vertreter: Urbach, Hans-Georg, Dr. et al,
Hanauer Landstrasse 526
D-6000 Frankfurt am Main 61(DE)

(54) **Tetrahydropyridazinonderivate, Verfahren zu ihrer Herstellung und ihre Verwendung.**

(57) Tetrahydropyridazinone der Formel I

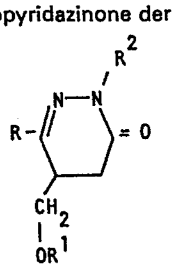

(I)

worin

R einen gegebenenfalls substituierten carbocyclischen oder heterocyclischen Rest,

$R^1$ Wasserstoff; Alkyl; Aralkyl, das im Arylteil gegebenenfalls substituiert ist; oder Acyl,

$R^2$ Wasserstoff; Alkyl, das gegebenenfalls substituiert sein kann; oder gegebenenfalls substituiertes Phenyl;

bedeuten, sowie ihre Säureadditionsverbindungen, soweit diese herstellbar sind.

Die erfindungsgemäßen Tetrahydropyridazinonderivate der Formel I und ihre physiologisch verträglichen Salze zeigen wertvolle pharmakologische Wirkungen.

EP 0 129 791 A2

0129791
Ref. 3275/3302
Dr-Va/St

# Tetrahydropyridazinonderivate, Verfahren zu ihrer Herstellung und ihre Verwendung

Die vorliegende Erfindung betrifft Tetrahydropyridazinone der Formel I

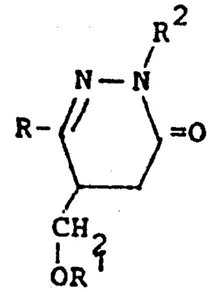

(I)

worin

R

einen der Reste der Formeln

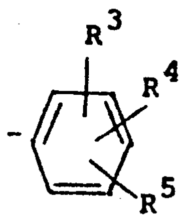

oder

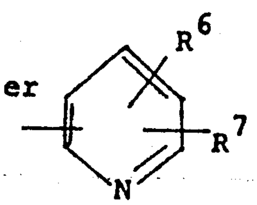

oder

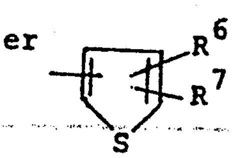

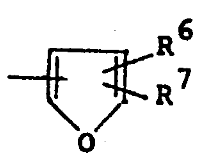

,

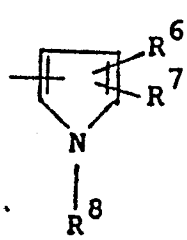

,

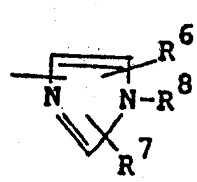

,

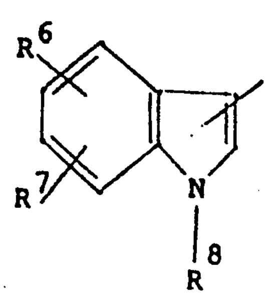

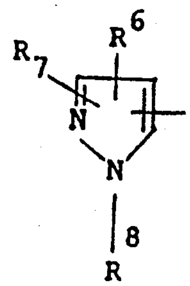

,

$R^1$ Wasserstoff; Alkyl; Aralkyl, das im Arylteil gegebenenfalls substituiert ist; oder Acyl der Formel $-CO-R^9$;

$R^2$ Wasserstoff; Alkyl, das gegebenenfalls durch basische Gruppen substituiert sein kann; oder gegebenenfalls substituiertes Phenyl;

$R^3$, $R^4$ und $R^5$ unabhängig voneinander Halogen; Alkyl mit 1 bis 5 C-Atomen; -OH; Alkoxy mit 1 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1$-$C_5)$-oxycarbonyl, eine Gruppe der Formel $-NR^{10}R^{11}$, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

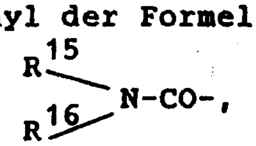

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3

Heteroatomen substituiert sein kann;
$R^4$ und $R^5$ zusätzlich auch Wasserstoff;
$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;
$R^8$ Wasserstoff, Alkyl mit 1 bis 5 C-Atomen, Alkanoyl mit 1 bis 5 C-Atomen oder ein Rest der Formel

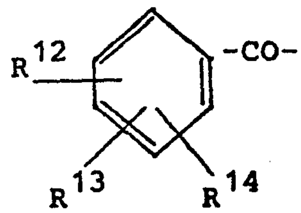

$R^9$ Alkyl mit 1 bis 5 C-Atomen, gegebenenfalls substituiert durch einen gegebenenfalls substituierten Phenoxyrest,

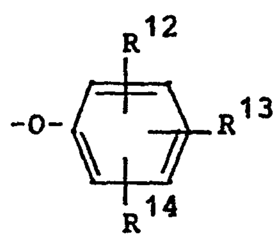

eine Gruppe der Formel

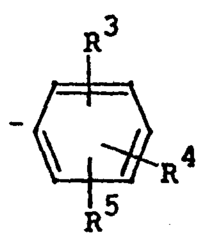

oder Pyridyl;
$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

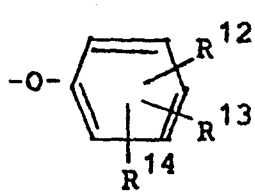

substituiert sein kann; Benzoyl der Formel

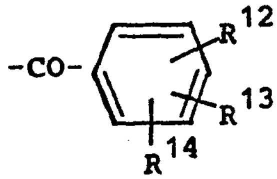

Heteroaryl der Formel

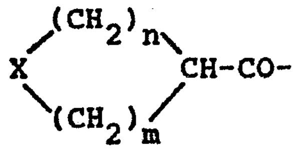

worin n = 2; 3 oder 4, m = 0; 1 oder 2 ist, wobei vorzugsweise n+m = 3 oder 4 ist, X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen, Chlor, Brom oder doppeltgebundenen Sauerstoff (=O) substituiert sein kann,

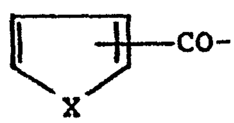 oder 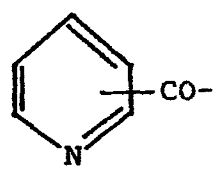 , und

$R^{11}$ Wasserstoff, Alkyl mit 1 bis 5 C-Atomen, Alkanoyl mit 1 bis 5 C-Atomen bedeuten oder $R^{10}$ und $R^{11}$ gemeinsam mit dem Stickstoffatom, an das sie gebunden sind, einen Pyrrolidin-, Piperidin-, Piperazin-, Morpholin- oder Thiomorpholinring oder einen Ring der Formeln

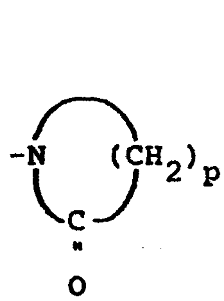

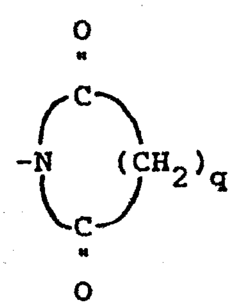

worin p = 3, 4 oder 5; q = 2 oder 3 ist,

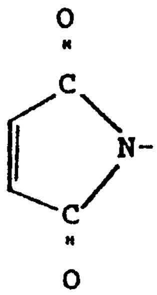 ; 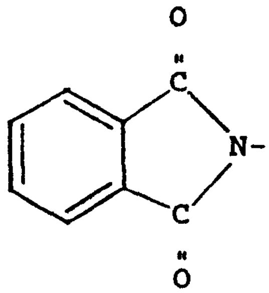 ; 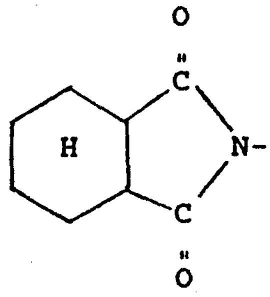 ;

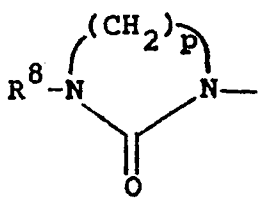 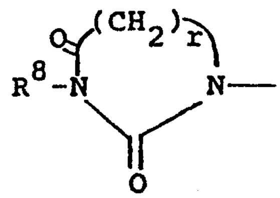

worin r = 1 oder 2 ist,

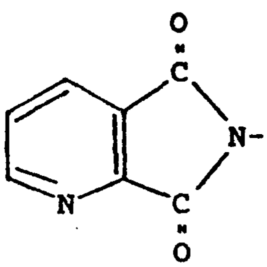 ; 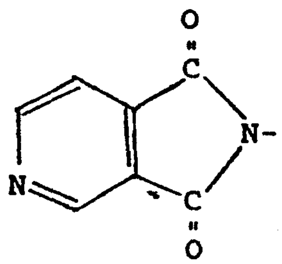 ; 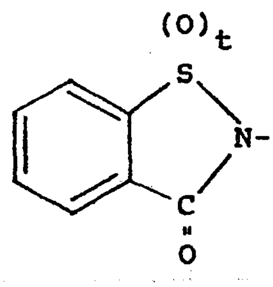

oder 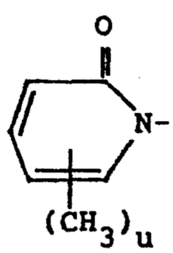

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen,

Alkanoylamino mit 1 bis 5 C-Atomen oder Benzoylamino bedeuten sowie ihre Säureadditionsverbindungen, soweit diese herstellbar sind.

Die erfindungsgemäßen Tetrahydropyridazinonderivate der Formel I, worin die Substituenten $R^3$, $R^4$, $R^5$ eines für R stehenden Phenylkerns die oben angegebenen Bedeutungen haben, und ihre physiologisch verträglichen Salze zeigen wertvolle pharmakologische Wirkungen. Sie sind den bisher bekannten Verbindungen gleicher Wirkungsrichtung überraschenderweise erheblich überlegen.

Ähnlich wertvolle pharmakologische Wirkung zeigen auch die Verbindungen der Formel I, in denen R ein unsubstituierter Phenylkern ist, worin also auch noch $R^3$ Wasserstoff bedeutet, und deren physiologisch verträgliche Salze.

Die Substituenten $R^3$ bis $R^5$ eines für R stehenden Restes der Formel

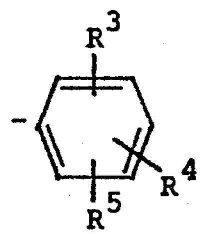

können in beliebigen Positionen des Phenylkerns stehen. Sind $R^4$ und $R^5$ Wasserstoff, so kann $R^3$ die oben genannten Bedeutungen haben und in 2-, 3- oder 4-Stellung des Phenylkerns stehen. Bei Zweifachsubstitution ist nur $R^5$ Wasserstoff, $R^3$ und $R^4$ haben unabhängig voneinander die oben genannten Bedeutungen und können in 2,3-; 3,2-; 2,4-; 4,2-; 2,5-; 5,2-; 2,6-; 3,4-; 4,3- oder 3,5-Stellung stehen. Vorzugsweise hat ein für R stehender, zweifachsubstituierter Phenylkern nur eine gegebenenfalls substituierte Aminogruppe und nur eine Acyloxygruppe, d.h. nur $R^3$ hat die oben definierten Bedeutungen $-NH_2$ Mono- oder Dialkylamino oder Alkanoyl- oder Benzoyloxy.

Ein evtl. vorhandener dritter Substituent ($R^5$ = Wasserstoff) ist vorzugsweise Chlor, ein Alkylrest, insbesondere Methyl, oder ein Alkoxyrest, insbesondere Methoxy.

5- oder 6-gliedrige Heterocyclen mit 1 bis 3 Heteroatomen, die als Substituent einer für $R^3$ oder $R^4$ stehenden Alkyl, Alkoxy-, Alkylamino-, Dialkylamino-, Alkylmercapto-, Alkylsulfoxy- oder Alkylsulfonylgruppe stehen können, sind aromatische und heteroaromatische Fünfringe mit 1, 2 oder 3 Stickstoffatomen, wie z.B. Pyrrol, Pyrrol-N-oxid, Pyrazol, Imidazol, s-Triazol, Pyrrolidin, Pyrrolidin-N-oxid, Pyrazolin, Imidazolin, s-Triazolin, Fünfringe mit einem Sauerstoffatom wie Furan und Tetrahydrofuran, mit einem Schwefelatom wie Thiophen, Tetrahydrothiophen, Thiophen-S-oxid, Tetrahydrothiophen-S-oxid, Tetrahydrothiophen-S-dioxid oder mit verschiedenen Heteroatomen wie Oxazol, Oxazolin, Thiazol, Thiazolin, Oxadiazol, Oxadiazolin oder aromatische und heteroaromatische Sechsringe mit 1, 2 oder 3 Stickstoffatomen wie Pyridin, Pyridin-N-oxid, Piperidin, Pyridazin, Di-, Tetra- und Hexahydropyridazin, Pyrazin, Di-, Tetra- und Hexahydropyrazin, Pyrimidin, Di-, Tetra-und Hexahydropyrimidin, s-Triazin, Di-, Tetra- und Hexahydro-s-triazin, Sechsringe mit einem oder zwei Sauerstoffatomen wie z.B. Pyran, Di- und Tetrahydropyran, 1,3- und 1,4-Dioxan, 1,3- und 1,4-Dioxen, 1,4-Dioxadien oder mit einem oder zwei Schwefelatomen wie Thiapyran und Tetrahydrothiapyran sowie deren S-Oxide und S-Dioxide, 1,4-Dithian, 1,4-Dithien und 1,4-Dithiadien sowie deren S-Oxide und S-Dioxide oder Sechsringe mit verschiedenen Heteroatomen wie z.B. 1,3- und 1,4-Oxazin in seinen tautomeren Formen, Dihydro-1,3- und -1,4-Oxazin, Morpholin, 1,3- und 1,4-Thiazin, Dihydro-1,3- und -1,4-Thiazin, Thiomorpholin, 1,4-Oxathian, 1,4-Oxathien und 1,4-Oxathiadien.

Die genannten Heterocyclen können ihrerseits noch einen oder zwei, vorzugsweise einen, Alkylrest mit 1 bis 5

C-Atomen, vorzugsweise 1 oder 2 C-Atomen, tragen. Sofern hydrierte oder teilhydrierte Formen vorliegen, können sie auch doppeltgebundenen Sauerstoff (Ketofunktionen) enthalten. Bei Stickstoffheterocyclen, die eine =NH-Gruppe im Ring enthalten, kann die Bindung an die für $R^3$ oder $R^4$ stehende Alkyl-, Alkoxy-, Alkylamino-, Dialkylamino-, Alkylmercapto-, Alkylsulfoxy- oder Alkylsulfonylgruppe auch über das N-Atom dieser =NH-Gruppe erfolgen. Soweit die eine Ketofunktion enthaltenden Stickstoffheterocyclen tautomere Formen bilden, können sie auch in dieser Form vorliegen.

Bevorzugte heterocyclische Reste sind z.B. Pyrrol, Pyrrolin, Pyrrolidin, Pyrrolidon, Imidazol-(1)-yl, Imidazol-4-yl, 2-Methyl-imidazol-4-yl, Oxazol, Oxazolin, Oxazolidin, Oxazolidon, insbesondere 2-Oxazolidon-5-yl, Oxadiazol-2-yl, 5-Methyl-oxadiazol-2-yl, Pyrid-2-, -3- oder -4-yl; 2-Hydroxy-4-methyl-pyrid-6-yl bzw. 2-Pyridon-4-methyl-6-yl, 2-Pyron-4-, -5-oder -6-yl, 2-Pyron-4-methyl-6-yl.

Für $R^{10}$ stehende Heteroarylreste der obigen Formel leiten sich von Carbonsäuren der Formel

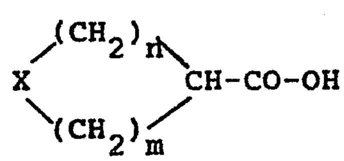

wie beispielsweise von der Pyrrolidin-2-carbonsäure, der in 1-Stellung durch $R^8$ substituierten Pyrrolidin-2-carbonsäure, der Pyrrolid-2-on-5-carbonsäure, deren in 1-Stellung durch $R^8$ substituiertem Derivat, der Pyridin-2-, -3- oder -4-carbonsäure, der Piperidin-2-, -3- oder -4-carbonsäure oder deren in 1-Stellung durch $R^8$ substituiertem Derivat, der Furan-2- oder -3-carbonsäure oder der Thiophen-2-oder -3-carbonsäure ab.

Reste der obigen Formel 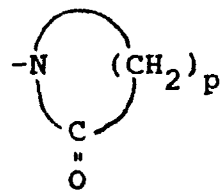

sind beispielsweise Pyrrolid-2-on-1-yl, Piperid-2-on-1-yl und der Caprolactamrest

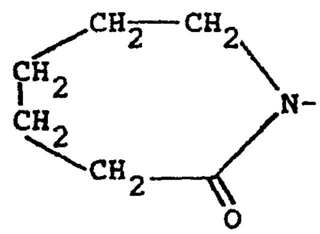

Reste der oben bereits angegebenen Formel

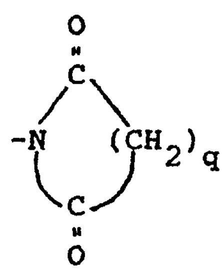

sind beispielsweise: der Succinimidrest

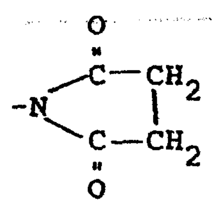

,

der Glutarimidrest

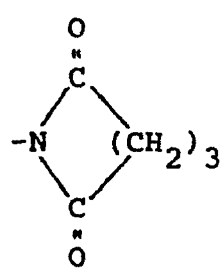

,

der Brenzweinsäureimidrest

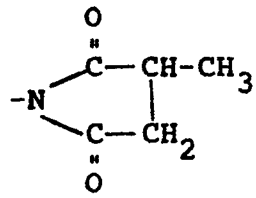

,

der symm. Dimethylbernsteinsäure-imidrest und der Pimelinsäure-imidrest.

Reste der Formeln

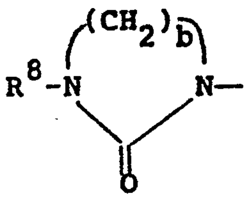

bzw.

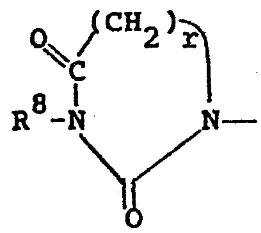

sind beispielsweise

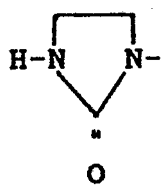

und

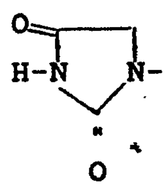

.

Sofern $R^{11}$ Wasserstoff ist, sind für $R^{10}$ folgende Bedeutungen bevorzugt: 1-Formyl-pyrrolidin-2-carbonyl, Pyrrolidin-2-carbonyl, 1-Acetyl-pyrrolidin-2-carbonyl, 5-Oxopyrrolidin-2-carbonyl, 2-Furylcarbonyl, 2,3,4-Pyridylcarbonyl, 2-Thienylcarbonyl, Benzoyl, o,m,p,-Methylbenzoyl, o,m,p-Methoxybenzoyl, 3,4-Dimethoxybenzoyl, o,m,p-Chlorbenzoyl, o,m,p-Cyanobenzoyl, o,m,p-Nitrobenzoyl, 4-Chlorphenoxyacetyl, Chloracetyl, Dichloracetyl, Aminoacetyl.

Ist $R^{11}$ nicht Wasserstoff, so sind die folgenden Kombinationen $R^{10}/R^{11}$ bevorzugt:

Acetyl/Acetyl, Acetyl/Alkyl($C_1$-$C_3$), Chloracetyl/Alkyl-($C_1$-$C_3$), Alkyl($C_1$-$C_5$)/Alkyl($C_1$-$C_5$), insbesondere Acetyl/-Methyl; Acetyl/Ethyl; Chloracetyl/Methyl; Chloracetyl/-Ethyl; Methyl/Methyl; Ethyl/Ethyl.

Bilden $R^{10}$ und $R^{11}$ gemeinsam einen an das N-Atom gebundenen, zweiwertigen Rest, so ist dieser vorzugsweise Butylen-1-carbonyl, Pentylen-1-carbonyl, Butylen-1,4-dicarbonyl, Butenylen-1,4-dicarbonyl, Phenylen-1,2-dicarbonyl, Phenylen-1-sulfonyl-2-carbonyl, Phenylen-1-sulfinyl-2-carbonyl, Phenylen-1-sulfenyl-2-carbonyl, Pyridyl-2,3-dicarbonyl.

Für die Stellung und Kombination der Substituenten $R^{12}$ bis $R^{14}$ eines Phenoxyrestes

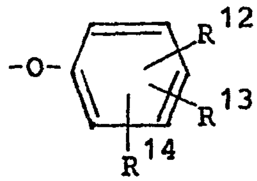

,

der Substituent einer für $R^{10}$ stehenden Alkanoylgruppe ist, sowie eines für $R^{10}$ stehenden Benzoylrestes der Formel

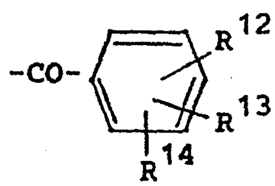

gelten die gleichen Regeln und Vorzugsbedingungen, wie sie für die Reste $R^3$ bis $R^5$ oben angegeben worden sind.

Bevorzugt sind solche für R stehenden Phenylreste, in denen $R^4$ und $R^5$ Wasserstoff bedeuten und $R^3$ eine der folgenden Bedeutungen hat:
o,m,p-Nitro, o,m,p-Cyano, o,m,p-Alkoxycarbonyl, 2-, 3- oder 4-Pyridylmethyl, 5-Methyl-(1,3,4-oxdiazol)-2-yl-methoxy, 2-, 3- oder 4-Pyridylmethoxy, (4-Methyl-2-pyron-6-yl)-methoxy, (4-Methyl-2-pyridon-6-yl)-methoxy, (2-Methyl-imidazol-4(5)-yl)-methoxy, 2-(1-Imidazolyl)-eth-

oxy, (Oxazolidin-2-on)-5-yl-methoxy, 2-(Methoxy)-ethoxy und 2-(Dimethylamino)-ethoxy. Bevorzugt sind auch solche Reste R, in denen $R^3/R^4/R^5$ in einer der folgenden Kombinationen vorliegen:
Alkyl/H/H; Fluor, Chlor oder Brom/H/H;
Alkoxy/H/H; Alkyl/Alkyl/H; Alkyl/Alkoxy/H;
Alkoxy/Alkoxy/H; Alkoxy/Alkoxy/Alkoxy;
Monoalkylamino/H/H; Dialkylamino/H/H; Acetylamino/H/H
sowie solche Reste R, in denen $R^3$ eine wie oben angegeben substituierte Alkyl- und insbesondere Alkoxygruppe ist, $R^4$ Wasserstoff oder Alkyl oder Alkoxy der oben angegebenen Kettenlänge, insbesondere Methyl oder Methoxy und $R^5$ Wasserstoff ist.

Beispiele für derartige bevorzugte für R stehende Reste sind Methylphenyl, insbesondere 4-Methylphenyl, Methoxyphenyl, insbesondere 4-Methoxyphenyl, Chlorphenyl, insbesondere 4-Chlorphenyl, Dimethoxyphenyl, insbesondere 3,4-Dimethoxyphenyl, Trimethoxyphenyl, insbesondere 3,4,5-Trimethoxyphenyl, Dimethylaminophenyl, insbesondere 4-Dimethylaminophenyl, 4-Acętylaminophenyl.

Weiterhin sind als Reste R bevorzugt:
2-Thienyl, 3-Pyridyl, 2-Furyl, 2-Pyrrolyl, 3-Indolyl, 4-(5-Amino-1,3-dimethyl-pyrazol-yl).

Ein für $R^1$ stehender Alkylrest kann geradkettig oder verzweigt sein und hat in der Regel 1 bis 5 C-Atome.

Bevorzugte für $R^1$ stehende Alkylreste sind geradkettig und haben 1 bis 5, insbesondere 1 oder 2, C-Atome.
Ein für $R^1$ stehender Aralkylrest ist vorzugsweise ein Phenalkylrest, der in der Alkyl-Brücke 1 bis 3, insbesondere 1 oder 2, C-Atome aufweist. Der Phenylkern eines für $R^1$ stehenden Phenalkylrestes ist unsubstituiert, er kann aber auch vorzugsweise einen oder zwei Substituenten tragen. Als Substituenten kommen hierbei in Betracht:

Halogen, insbesondere Fluor, Chlor oder Brom, Alkyl mit 1 bis 5, vorzugsweise 1 oder 2, C-Atomen, Alkoxy mit 1 bis 5, vorzugsweise 1 oder 2, C-Atomen, die Aminogruppe, Monoalkylamino mit 1 bis 5, vorzugsweise 1 oder 2, C-Atomen, Dialkylamino mit insgesamt 2 bis 6, vorzugsweise 2 bis 4, C-Atomen.

Die Substituenten des Phenylkerns eines für $R^1$ stehenden Phenylalkylrestes können in beliebigen Positionen stehen. Ein einzelner Substituent kann in 2-, 3- oder 4-Stellung des Phenylkerns stehen.

Bei Zweifachsubstitution können die Substituenten in 2,3-; 3,2-; 2,4-; 4,2-; 2,5-; 5,2-; 2,6-; 3,4-; 4,3- oder 3,5-Stellung stehen. Vorzugsweise hat ein im Phenylkern zweifach substituierter Phenalkylrest nur eine gegebenenfalls substituierte Aminogruppe. Ein evtl. vorhandener zweiter Substituent ist vorzugsweise Chlor, ein Alkylrest, insbesondere Methyl oder ein Alkoxyrest, insbesondere Methoxy.

Steht $R^1$ für einen Alkanoylrest der Formel $-CO-R^9$, so kann der Alkylrest $R^9$ ebenfalls geradkettig oder verzweigt sein. In der Regel hat dieser Alkylrest 1 bis 5, insbesondere 1 oder 2, C-Atome.

Steht $R^1$ für einen Acylrest der Formel $-CO-R^9$ und $R^9$ für Pyridyl, so kann der Pyridylrest in 2-, 3- oder 4-Stellung gebunden sein.

Ein Phenoxyrest, der als Substituent an eine für $R^9$ stehende Alkylgruppe gebunden ist, kann unsubstituiert oder durch Halogen, vorzugsweise Chlor, Alkyl mit 1 bis 4 C-Atomen, vorzugsweise Methyl oder Alkoxy mit 1 bis 4 C-Atomen, vorzugsweise Methoxy, substituiert sein. Besonders bevorzugt sind erfindungsgemäße Verbindungen, in denen $R^1$ für Wasserstoff; Alkyl mit 1 oder 2 C-Atomen,

insbesondere Methyl; Alkanoyl mit 1 bis 3 C-Atomen, insbesondere Acetyl, Benzoyl oder Nicotinoyl steht.

Ein für $R^2$ stehender Alkylrest kann geradkettig oder verzweigt sein und hat in der Regel 1 bis 5 C-Atome. Basische Gruppen, durch die eine für $R^2$ stehende Alkylgruppe gegebenenfalls substituiert sein kann, entsprechen der Formel

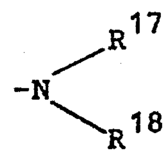

worin $R^{17}$ und $R^{18}$ unabhängig voneinander Wasserstoff; Alkyl mit 1 bis 6 C-Atomen; Cycloalkyl mit 3 bis 6 C-Atomen bedeuten oder worin $R^{17}$ und $R^{18}$ gemeinsam für eine Polymethylenkette mit 2 bis 5 C-Atomen stehen, die gegebenenfalls durch ein Sauerstoff- oder Schwefelatom oder durch eine Gruppe $=N-R^{19}$ unterbrochen sein kann, wobei $R^{19}$ Wasserstoff; Alkyl mit 1 bis 4 C-Atomen, vorzugsweise Methyl, oder Phenalkyl mit 1 bis 3 C-Atomen in der Alkylbrücke, vorzugsweise Benzyl, bedeutet.

Ein für $R^2$ stehender Phenylrest kann unsubstituiert sein, oder er kann bis zu drei, vorzugsweise zwei, und insbesondere einen der folgenden Substituenten tragen: Halogen; Alkyl mit 1 bis 5 C-Atomen; -OH; Alkoxy mit 1 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; und Alkanoylamino mit 1 bis 4 C-Atomen, wobei der Alkylrest mit 1 bis 5 C-Atomen seinerseits durch $-NH_2$; Monoalkylamino mit 1 bis 5 C-Atomen; Dialkylamino mit insgesamt 2 bis 5 C-Atomen; und Alkanoylamino mit 1 bis 4 C-Atomen substituiert sein kann.

Im übrigen gelten für Anzahl und Stellung dieser Substituenten dieselben Vorzugsregeln, die oben bezüglich der Substitution eines für R stehenden Phenylrestes angegeben

wurden.

Besonders bevorzugt sind solche erfindungsgemäßen Tetrahydropyridazinonderivate, in denen mehrere der oben genannten bevorzugten Merkmale vereinigt sind.

Bevorzugt sind auch erfindungsgemäße Tetrahydropyridazinone, in denen $R^1$ Wasserstoff ist, und ferner solche, in denen $R^2$ Wasserstoff ist, und insbesondere solche, in denen $R^1$ und $R^2$ Wasserstoff sind. Diese Gruppen erfindungsgemäßer Verbindungen sind, abgesehen von ihrer guten pharmazeutischen Wirksamkeit, auch als Zwischenprodukte zur weiteren Umsetzung mit Alkylierungsmitteln bzw. Acylierungsmitteln zu erfindungsgemäßen Verbindungen, in denen $R^1$ und/oder $R^2$ von Wasserstoff verschiedene Bedeutungen haben, einzusetzen.

Zur Salzbildung sind prinzipiell alle anorganischen und organischen Säuren geeignet, deren Stärke ausreicht, die erfindungsgemäßen Verbindungen zu protonieren. Die Auswahl kann somit in an sich bekannter Weise erfolgen. Beispiele für geeignete Säuren sind HCl, HBr, $H_2SO_4$, Phosphorsäure, Ameisensäure, Essigsäure, Propionsäure, Milchsäure, Oxalsäure, Fumarsäure, Weinsäure, Benzoesäure, Salicylsäure, Methansulfonsäure.

Bevorzugte Salze erfindungsgemäßer Verbindungen sind solche, an denen pharmakologisch akzeptable Säuren beteiligt sind.

Die erfindungsgemäßen Tetrahydropyridazinonderivate und ihre Salze weisen wertvolle pharmakologische Wirkungen auf.

Die Herstellung der erfindungsgemäßen Tetrahydropyridazinonderivate erfolgt durch Umsetzung von Acyl-γ-butyrolactonen der Formel II mit Hydrazin oder Hydrazinderivaten der Formel III nach dem Schema

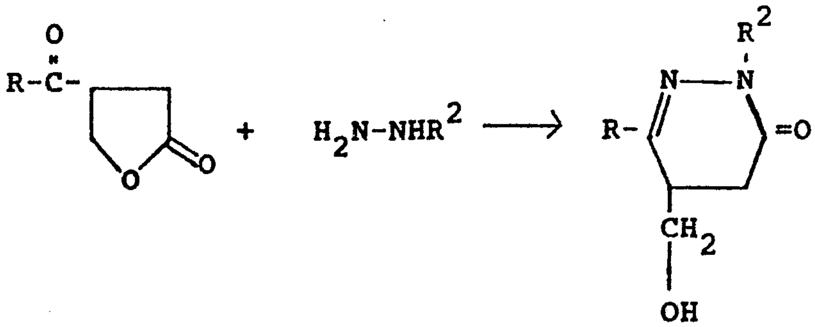

(II) (III) (Ia)

worin R und $R^2$ die obengenannten Bedeutungen haben, und, sofern erfindungsgemäße Verbindungen hergestellt werden sollen, in denen $R^1$ von Wasserstoff verschieden ist, anschließende Verätherung bzw. Veresterung der 5-ständigen Hydroxymethylgruppe mit einem Verätherungs- bzw. Veresterungsmittel der Formel IV

$$R^1 - X \qquad (IV)$$

worin $R^1$ die obengenannte Bedeutung hat und X ein als Anion abspaltbarer Rest ist.

Die Herstellung der oben angegebenen erfindungsgemäßen Verbindungen der Formel Ia kann auch in zwei unabhängigen Schritten erfolgen, indem zunächst das Butyrolactonderivat II mit Hydrazin ($NH_2$-$NH_2$) umgesetzt wird zu dem erfindungsgemäßen Tetrahydropyridazinonderivat Ib

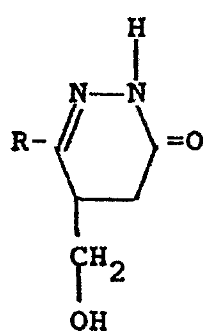

(Ib)

das gewünschtenfalls in der 2-Stellung in an sich bekannter Weise alkyliert werden kann. Dieses Verfahren ist dann von Vorteil, wenn entsprechend substituierte Hydrazine der Formel III schwerer zugänglich sind.

Der erste Reaktionsschritt bei der Herstellung der erfindungsgemäßen Tetrahydropyridazinonderivate, die Umsetzung der Acyl-γ-butyrolactone der Formel II mit Hydrazinen der Formel III, beinhaltet die Umsetzung einer γ-Ketocarbonsäure bzw. eines γ-Ketocarbonsäurederivats mit Hydrazin unter Cyclisierung zu dem korrespondierenden Tetrahydropyridazinonderivat. Diese Reaktion sowie die Reaktionsbedingungen sind als solche seit langem bekannt. So ist bereits von E. Fischer in " Liebigs Annalen der Chemie", Band 236, S. 147 im Jahre 1886 über die Umsetzung von Lävulinsäure (γ-Ketovaleriansäure) mit Phenylhydrazin zu 2-Phenyl-5-methyl-tetrahydropyridazinon-(3) berichtet worden. Weitere Angaben über diese Reaktion finden sich in Liebigs Annalen der Chemie, Bd 724, Seite 217 - 220 (1969).

Zweckmäßigerweise wird die Reaktion in flüssiger Phase durchgeführt, wozu in der Regel die Anwesenheit eines inerten Lösungs- bzw. Verdünnungsmittel erforderlich ist. Als Lösungsmittel eignet sich in vielen Fällen Wasser, insbesondere aber werden organische Lösungsmittel verwendet aus der Reihe der Alkanole; Alkandiole; Diglycol oder Triglycol sowie deren Äther und Halbäther; Dialkyläther; cyclische Äther, wie z.B. Tetrahydrofuran oder Dioxan; stark polare aprotische Lösungsmittel, wie beispielsweise DMF, DMSO oder Pyridin; aromatische Kohlenwasserstoffe, wie Benzol, Toluol, Xylol; aliphatische oder aromatische Halogenkohlenwasserstoffe wie Methylenchlorid, Chloroform, Trichlorethylen, Monochlor-benzol, Dichlorbenzol.

Die Reaktion kann prinzipiell bei Temperaturen zwischen Raumtemperatur und dem Siedepunkt des jeweils verwendeten Lösungsmittels durchgeführt werden. Bei niedrig siedenden Lösungsmitteln kann auch zur Beschleunigung der Cyclisierung im geschlossenen System unter Druck bei einer Temperatur oberhalb des Siedepunktes des Lösungsmittels gearbeitet werden. In der Regel wird die Reaktion nicht oberhalb 200°C durchgeführt. Bevorzugt arbeitet man bei einer Reaktionstemperatur zwischen 50 und 100°C. Auch durch Säurekatalyse kann eine Reaktionsbeschleunigung erfolgen. Wird die Reaktion bei tiefer Temperatur durchgeführt, so lassen sich die intermediär entstehenden Hydrazone der Formel IIa, gegebenenfalls als Hydrate, isolieren,

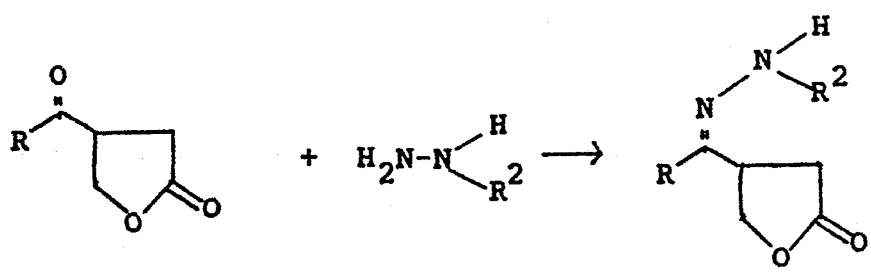

(II) (IIa)

die dann durch Erwärmen, gegebenenfalls unter Säurekatalyse, wie oben beschrieben, zu den Tetrahydropyridazinonen der Formel Ia umgesetzt werden können.

Die im ersten Reaktionsschritt erhaltenen erfindungsgemäßen Verbindungen der Formel Ia weisen in der 5-Stellung des Tetrahydropyridazinon-Systems eine Hydroxymethylgruppe auf, die in einem zweiten Reaktionsschritt gewünschtenfalls veräthert oder verestert werden kann. Verätherungen oder Veresterungen von OH-Gruppen, die an aliphatische Reste gebunden sind, sind in unübersehbarer Anzahl bereits beschrieben worden, und die Bedingungen dieser Reaktionen sind somit bestens bekannt. Ein guter Überblick über die hierzu zweckmäßigen Reaktionsbedingungen und Reagenzien findet sich beispielsweise in Houben Weyl Bd. 6/3, Seite 22ff und Houben Weyl Bd. 8, Seite 516ff; Synthesis (1982) S. 833; J. Med. chem. Bd. 25, Seite 620 (1982).

Im Rahmen der vorliegenden Erfindung kann eine gegebenenfalls gewünschte Verätherung der 5-ständigen Hydroxymethylgruppe durch Erwärmen der Verbindung der Formel Ia mit einem Alkylierungsmittel der Formel IV durchgeführt werden. In dem Alkylierungsmittel der Formel $R^1X$ bedeutet X einen als Anion abspaltbaren Rest. Solche Reste sind in großer Zahl ebenfalls aus der Literatur bekannt.

Beispiele hierfür sind Halogenatome, eine Alkylsulfatogruppe oder beispielsweise Arylsulfonyloxy. Je nach Art des Restes X ist es zweckmäßig, den Fortschritt der Reaktion durch Zugabe eines geeigneten Katalysators, bzw. einer Base zu begünstigen.
Geeignete Basen sind beispielsweise tertiäre Amine, vorzugsweise aber anorganische Basen wie Alkalihydroxyde oder Salze von Alkalimetallen mit schwachen anorganischen oder organischen Säuren, insbesondere zum Beispiel die Karbonate der Alkalimetalle. Auch die Alkylierungsreaktion wird zweckmäßigerweise in einem Lösungsmittel durchgeführt, vorzugsweise einem organischen Lösungsmittel oder aber in einem Überschuß des Alkylierungsmittels. Lösungsmittel, die für die Alkylierung in Betracht kommen, sind beispielsweise Äther, insbesondere cyclische Äther, wie Tetrahydrofuran oder Dioxan, aromatische Kohlenwasserstoffe, wie zum Beispiel Benzol, Toluol oder Xylol.

Eine evtl. gewünschte Veresterung der 5-ständigen Hydroxy-methylgruppe läßt sich ebenfalls in an sich bekannter Weise durchführen. Als Veresterungsmittel der Formel $R^1X$ können mit besonderem Vorteil die Carbonsäure-imidazolide eingesetzt werden.

Andere geeignete Veresterungsmittel sind solche, in denen X beispielsweise für den Alkanoyloxyrest steht. In diesem Fall werden Karbonsäureanhydride eingesetzt. Die Veresterungsreaktion kann im Prinzip ohne jedes Lösungsmittel durchgeführt werden, es ist jedoch insbesondere bei der Verwendung reaktiver Veresterungsmittel zweckmäßig, zur Moderierung der Reaktion in einem Lösungs- oder Verdünnungsmittel zu arbeiten. Geeignet sind alle organischen Lösungsmittel, die mit den Acylierungsmitteln nicht in Reaktion treten können. Vorwiegend verwendet man aromatische Kohlenwasserstoffe, wie Benzol, Toluol, Xylol als Lösungs-, Verdünnungs- oder Dispergiermittel, wobei, insbesondere, wenn nicht die Salze erfindungsgemäßer Tetrahydropyridazinone, sondern die freien Basen hergestellt werden sollen, es zweckmäßig ist, die Acylierung ebenfalls in Gegenwart eines basischen Säurefängers, wie beispielsweise einer tertiären organischen Aminbase, vorzunehmen. Acylierungen der genannten Art können auch in Gegenwart von Pyridin- oder Pyridinabkömmlingen als Lösungsmittel durchgeführt werden. In diesem Fall wirkt das Lösungsmittel selbst als Säurefänger. Auch die Acylierung der erfindungsgemäßen Verbindungen der Formel Ia kann bei Temperaturen zwischen der normalen Zimmertemperatur und Temperaturen bis 150°C ausgeführt werden. Höhere Temperaturen bringen in der Regel keine Vorteile mehr. Besonders zweckmäßig ist die Durchführung der Acylierung im Bereich unterhalb des Siedepunkts des eingesetzten Lösungs- oder Dispergiermittels, insbesondere bei Temperaturen zwischen Zimmertemperatur und 60°C.

Nach dem oben angegebenen Reaktionsschema wird der Rest $R^2$ in die erfindungsgemäßen Verbindungen dadurch eingeführt, daß zur Umsetzung mit dem Ketocarbonsäurederivat ein durch $R^2$ substituiertes Hydrazin eingesetzt wird. Wie bereits oben gesagt, ist es jedoch auch möglich, den Rest $R^2$ in eine erfindungsgemäße Verbindung der Formel Ib

nachträglich einzuführen. Die Einführung eines Restes $R^2$ in der 2-Stellung erfindungsgemäßer Verbindungen der Formel Ib stellt eine Alkylierung bzw. Arylierung unter Einsatz eines Alkylierungs- bzw. Arylierungsmittels der der Formel V

$$R^2 X \qquad (V)$$ dar.

N-Alkylierungen an heterocylischen Verbindungen sind ebenfalls seit langem Stand der Technik und in zahlreichen Publikationen, wie beispielsweise "Heterocyclic Compounds" Vol. 28, Seite 37ff. (1973), eingehend beschrieben worden. Auch in dem Alkylierungsagenz $R^2X$ bedeutet X einen als Anion abspaltbaren Rest, vorzugsweise ein Halogenatom, eine Alkylsulfatogruppe oder Arylsulfonyloxy. Es ist zweckmäßig, den Fortschritt der Reaktion durch Zugabe eines geeigneten Katalysators bzw. einer den als Anion abgespaltenen Rest X abfangenden Base zu begünstigen. Geeignete Basen sind beispielsweise tertiäre Amine, vorzugsweise aber anorganische Basen,wie Alkalihydroxyde oder Salze von Alkalimetallen mit schwachen anorganischen oder organischen Säuren, insbesondere zum Beispiel die Karbonate der Alkalimetalle. Auch diese Alkylierungsreaktion wird zweckmäßigerweise in einem Lösungsmittel durchgeführt, z.B. in Wasser, oder in einem organischen Lösungsmittel. Lösungsmittel, die für die Alkylierung in Betracht kommen, sind beispielsweise Äther, insbesondere cyclische Äther, wie Tetrahydrofuran oder Dioxan, aromatische Kohlenwasserstoffe, wie zum Beispiel Benzol, Toluol oder Xylol.

Acyl-γ-butyrolactone der Formel II, worin R einen der Reste der Formeln

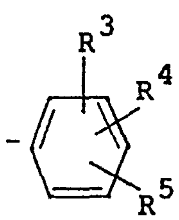 oder 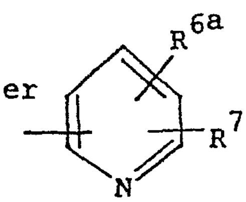 oder 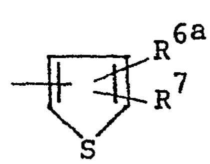

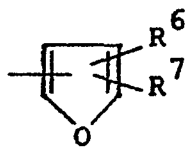 , 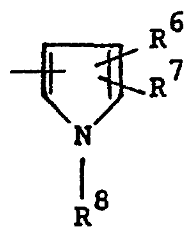 , 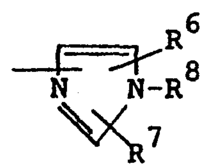 ,

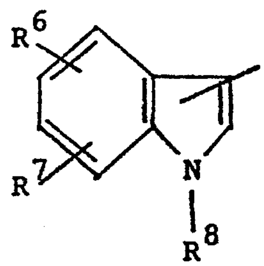 , 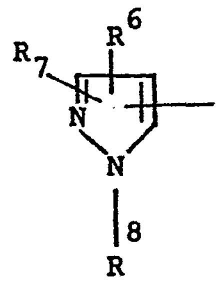

$R^3$, $R^4$ und $R^5$ unabhängig voneinander Jod, Alkyl mit 3 bis 5 C-Atomen, -OH; Alkoxy mit 2 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^4$ und $R^5$ zusätzlich auch Wasserstoff; einer der Reste $R^3$, $R^4$ oder $R^5$ o- oder m-Methoxy, o- oder m-Methyl oder -Ethyl, o- oder m-Brom, o-Fluor und o-Chlor, die beiden anderen Wasserstoff; zwei der Reste $R^3$, $R^4$ oder $R^5$ 2,3-, 2,4-, 2,5- 2,6-, 3,5-Dimethoxy, -Dichlor oder-Difluor oder 3,4-Difluor und der dritte Wasserstoff, oder alle drei der Reste $R^3$, $R^4$ und $R^5$ 2,3,4-; 2,3,5-; 2,3,6-; 2,4,5- oder 2,4,6-Trimethoxy; und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1-C_5)$-oxycarbonyl, eine Gruppe der Formel $-NR^{10}R^{11}$, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2,

C-Atomen, Carboxyl, Alkoxycarbonyl mit insgesamt 2 bis 7 C-Atomen, Aminocarbonyl der Formel

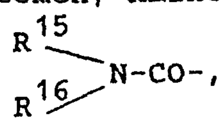

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3 Heteroatomen substituiert sein kann;

$R^4$ und $R^5$ zusätzlich auch Wasserstoff;

$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;

$R^{6a}$ und $R^7$ unabhängig voneinander Halogen, insbesondere Chlor und Brom; Amino, Monoalkylamino mit 1 bis 5 C-Atomen, Dialkylamino mit insgesamt 2 - 6 C-Atomen, Hydroxy, Alkoxy mit 1 bis 5 C-Atomen oder Alkyl mit 1 bis 5 C-Atomen; und, sofern Pyridyl in 2- oder 4-Position und Thienyl in 3-Position gebunden sind, zusätzlich Wasserstoff,

$R^8$ Wasserstoff, Alkyl mit 1 bis 5 C-Atomen, Alkanoyl mit 1 bis 5 C-Atomen oder ein Rest der Formel

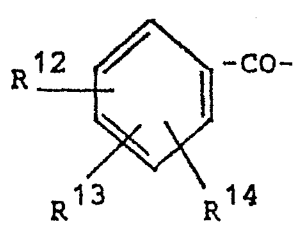

$R^9$ Alkyl mit 1 bis 5 C-Atomen, gegebenenfalls substituiert durch einen gegebenenfalls substituierten Phenoxyrest,

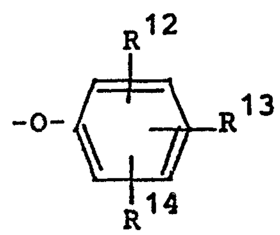

eine Gruppe der Formel

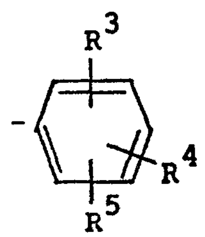

oder Pyridyl;
$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

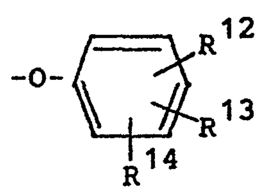

substituiert sein kann; Benzoyl der Formel

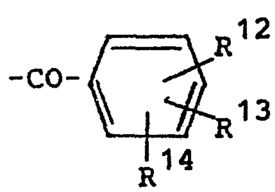

Heteroaryl der Formel

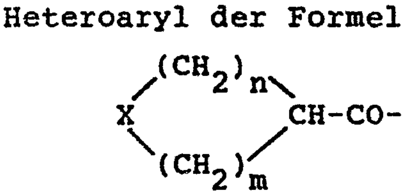

worin n = 2; 3 oder 4,
m = 0; 1 oder 2 ist, wobei vorzugsweise n+m = 3 oder 4 ist,
X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen,

Chlor, Brom oder doppeltgebundenen Sauerstoff (=O) substituiert sein kann,

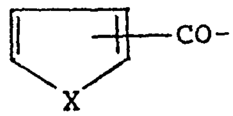 oder 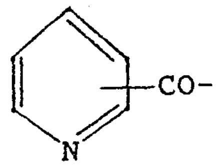, und

$R^{11}$ Wasserstoff, Alkyl mit 1 bis 5 C-Atomen, Alkanoyl mit 1 bis 5 C-Atomen bedeuten
oder $R^{10}$ und $R^{11}$ gemeinsam mit dem Stickstoffatom, an das sie gebunden sind, einen Pyrrolidin-, Piperidin-, Piperazin-, Morpholin- oder Thiomorpholinring oder einen Ring der Formeln

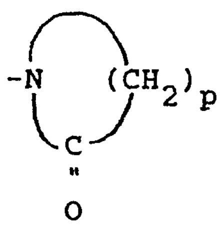

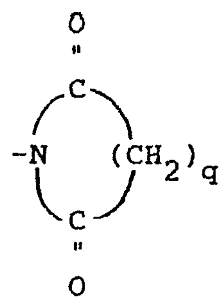

worin p = 3, 4 oder 5; q = 2 oder 3 ist;

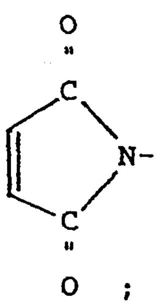 ; 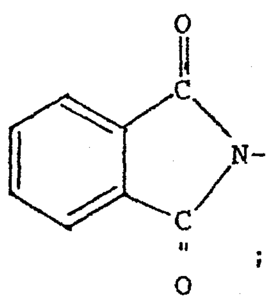 ; 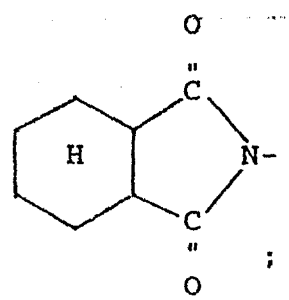 ;

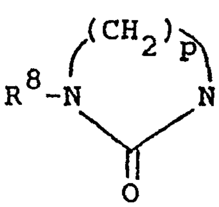

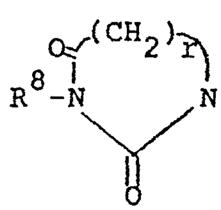

worin r = 1 oder 2 ist,

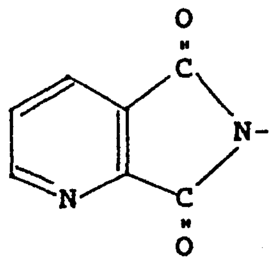 ; 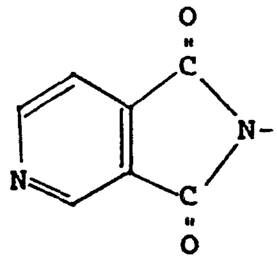 ; 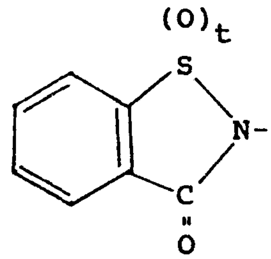

oder 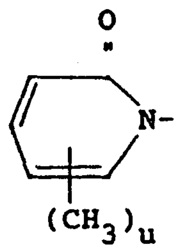

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen, Alkanoylamino mit 1 bis 5 C-Atomen oder Benzoylamino bedeuten, sind bisher nicht beschrieben worden und somit als Zwischenprodukte zur Herstellung der erfindungsgemäßen Tetrahydropyridazinone der Formel I ebenfalls Gegenstand der Erfindung.

Die als Ausgangsmaterialien für die Herstellung erfindungsgemäßer Tetrahydropyridazinonderivate eingesetzten Acyl-$\gamma$-butyrolactone der Formel II lassen sich aus in 4-Stellung substituierten 4-Ketobuttersäuren der Formel VI durch Umsetzung mit Formaldehyd herstellen:

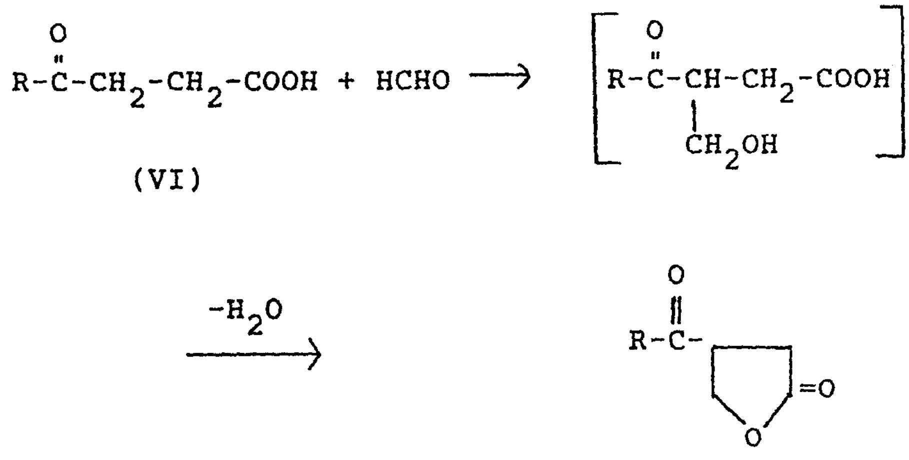


(VI)

(II)

Die Bedeutungen von R sind in den Formeln II und VI gleich. Oxymethylierungen von CH-Acidverbindungen durch Umsetzung mit Formaldehyd sind ebenfalls bereits Stand der Technik (vgl. hierzu J. Org. Chem. 24, S. 586-89 (1959); Synthesis (1980) S. 825-828.

Die Reaktion kann mit oder ohne Lösungsmittel und unter Verwendung von wäßrigem Formaldehyd, Paraformaldehyd, oder eines in einem organischen Lösungsmittel gelösten Formaldehyds durchgeführt werden. Die Umsetzung erfolgt in der Regel in Gegenwart eines schwach basischen Katalysators, wie z.B. Ammonacetat- oder Piperidinacetat. Beim Arbeiten im wässrigen Medium werden als basische Katalysatoren zweckmäßigerweise Hydroxyde oder basisch reagierende Salze von Alkali- oder Erdalkalimetallen eingesetzt. Die Hydroxymethylierung verläuft in der Regel bereits bei Zimmertemperatur mit beachtlicher Geschwindigkeit. Die Reaktion kann daher bei Temperaturen zwischen 20 und 100°C ausgeführt werden. Nach erfolgter Hydroxymethylierung wird das Reaktionsgemisch angesäuert und einige Stunden bei Temperaturen zwischen 20 und 120°C vorzugsweise bei Zimmertemperatur, nachgerührt. Hierbei

tritt Ringschluß der gebildeten Hydroxymethyl-γ-Ketobuttersäure zu dem Butyrolactonderivat der Formel II ein.

Prinzipiell ist es auch möglich, nach dem Ansäuern die Hydroxymethyl-γ-ketobuttersäure in freier, nichtringgeschlossener Form zu isolieren und mit Hydrazin oder einem Hydrazinderivat $R^2$-NH-$NH_2$ zu den erfindungsgemäßen Tetrahydropyridazinonderivaten umzusetzen. (Vgl. E. Fischer, Liebigs Annalen der Chemie).

γ-Ketobuttersäurederivate der Formel VI, die zur Herstellung der Acyl-γ-butyrolactone der Formel II verwendet werden, können auf verschiedenen bekannten Wegen erhalten werden. Unabhängig von der Natur des Restes R führt die Addition von Aldehyden der Formel R-CHO an Acrylsäure oder Acrylsäurederivate, insbesondere Acrylsäureester in guten Ausbeuten zu den gewünschten γ-Keto-buttersäuren, bzw. -buttersäurederivaten. Diese Additionsreaktion ist 1976 von H. Stetter in "Angewandte Chemie", Heft 21, Seite 695ff., beschrieben worden. Die Reaktion wird durch die gleichen Katalysatoren katalysiert, die für die bekannte Benzoin-Kondensation geeignet sind. Insbesondere kommen hierfür Cyanid-Ionen und quartäre Thiazoliumsalze in Betracht. Als Lösungsmittel für die Durchführung dieser Additionsreaktionen eignen sich polare Lösungsmittel, wobei sich niedere Alkanole wie Methanol oder Ethanol und Dimethylformamid besonders bewährt haben. Bereits bei Zimmertemperatur erreicht die Additionsreaktion eine beachtliche Geschwindigkeit, die durch Erwärmen noch gesteigert werden kann. Zweckmäßigerweise arbeitet man zwischen Raumtemperatur und 60°C ,vorzugsweise zwischen 30 und 50°C. Beispiele für Aldehyde, die in der angegebenen Weise mit Acrylsäure oder Acrylsäurederivaten, insbesondere Acrylsäureestern, umgesetzt werden können, sind Benzaldehyd, 2-, 3- oder 4-Chlorbenzal- dehyd; 2,4- oder 3,4-Dichlorbenzaldehyd; 4-Brombenzaldehyd, 2-, 3- oder 4-Methyl- oder -Ethylbenzaldehyd; 4-Isopropyl-benzaldehyd, 2-, 3- oder 4-Hydroxybenzaldehyd, 2,4-Dihydroxybenzaldehyd, 2-, 3- oder 4-Methoxy- oder -Ethoxybenzaldehyd, 2,4-, 3,4- oder 3,5-Dimethoxybenzaldehyd, 3,4,5-Trimethoxybenzaldehyd, p-Acetoxybenzaldehyd, p-Benzyloxybenzaldehyd, 2-, 3- oder 4-Methylmerkaptobenzaldehyd, 2-, 3- oder 4-Amino-, -Monomethylamino- oder -Dimethylaminobenzaldehyd, 2-, 3- oder 4-Nitrobenzaldehyd, 3-Methyl-, 3-Ethyl- oder 3-Butylpyridin-2-aldehyd, 4-, 5- oder 6-Methylpyridin-2-aldehyd, 4-Chlorpyridin-2-aldehyd, 5-Nitropyridin-2-aldehyd, 4-Chlor-6-methylpyridin-2-aldehyd, 4,6-Dichlorpyridin-2-aldehyd, 4,6-Dimethylpyridin-2-aldehyd, 6-Methyl-4-nitropyridin-2-aldehyd, 5-Brom- oder 5-Chlor-pyridin-3-aldehyd, 2- oder 6-Methylpyridin-3-aldehyd, 5,6-Dichlorpyridin-3-aldehyd, 2-Methylpyridin-4--aldehyd, 3-Ethylpyridin-4-aldehyd, 2-Methyl-5-ethylpyridin-4-aldehyd, 2,6-Dichlorpyridin-4-aldehyd, 2,6-Dimethylpyridin-4-aldehyd, 3-Nitropyridin-4-aldehyd, 5-Chlor- oder 5-Brom-thiophen-2-aldehyd, 3- oder 4-Bromthiophen-2-aldehyd, 4,5-Dichlor-oder 4,5-Dibromthiophen-2-aldehyd, 4- oder 5-Nitrothiophen-2-aldehyd, 3-Brom-4-Nitrothiophen-2-aldehyd, 5-Methyl-, -ethyl-, -butyl- oder -isobutyl-thiophen-2-aldehyd, 3-Methyl-5-Nitrothiophen-2-aldehyd, 2-Chlor-, 2-Brom- oder 2-Nitrothiophen-3-aldehyd, 2,5-Dichlorthiophen-3-aldehyd, 2,5-Dimethyl- oder 2,5-Diethylthiophen-3-aldehyd, 2-Butyl-5-methylthiophen-3-aldehyd, 5-tert. Butyl-2-methylthiophen-3-aldehyd.

Ist R ein substituierter Phenyl- oder Heteroarylrest, so können die -Ketobuttersäurederivate der Formel VI auch dadurch erhalten werden, daß man entsprechend substituierte Benzolderivate oder Heterocyclen unter Friedel-Crafts-Bedingungen mit Bernsteinsäureanhydrid oder Bernsteinsäure-ester-chlorid acyliert. Friedel-Crafts-Reaktionen mit Bernsteinsäureanhydrid sind ebenfalls seit langem Stand der Technik und in zahlreichen Publikationen, beispielsweise in J. Am. Chem. Soc. 70, S. 3197 (1948); Liebigs Annalen d. Chemie, 462, S. 148 (1928);

US Patent No. 2,447,998, detailliert dargestellt worden.

Bei Acylierung nach Friedel-Crafts werden die Reaktanten in einem inerten Lösungsmittel, vorzugsweise einem inerten aromatischen Kohlenwasserstoff, oder auch in Schwefelkohlenstoff in Gegenwart eines Friedel-Crafts-Katalysators bei Temperaturen zwischen Raumtemperatur und dem Siedepunkt des Lösungsmittels miteinander umgesetzt. Verwendet man Schwefelkohlenstoff als Lösungsmittel, so muß für ausreichende Kühlung Sorge getragen werden, damit das Lösungsmittel nicht verdampft. Beim Einsatz von Nitrobenzol, einem sehr gut geeigneten Lösungsmittel für die Durchführung von Friedel-Crafts-Reaktionen, sollte bekanntlich nicht oberhalb $50^{o}C$ gearbeitet werden, da es sonst zu heftigen Reaktionen kommen kann. Sollen -Ketobuttersäuren der Formel VI hergestellt werden, in denen R beispielsweise Chlorphenyl, Alkylphenyl oder Dialkylphenyl ist, so kann man in einfacher Weise Bernsteinsäureanhydrid mit einem Friedel-Crafts-Katalysator in dem entsprechend substituierten Benzolderivat, welches in größerem Überschuß eingesetzt wird und damit gleichzeitig als Lösungsmittel wirkt, umsetzen. Als Friedel-Crafts-Katalysator werden bekanntlich in der Regel Lewissäuren, wie $AlCl_3$, $SbCl_5$, $BF_3$, $ZnCl_2$, oder Protonensäuren, wie z.B. HF, $H_2SO_4$, $H_3PO_4$ beziehungsweise $P_2O_5$, eingesetzt. Vorzugsweise arbeitet man mit Aluminiumchlorid als Friedel-Crafts-Katalysator.

Die Herstellung der Säureadditionssalze der erfindungsgemäßen Tetrahydropyridazinone erfolgt in bekannter Weise durch einfaches Vereinigen der erfindungsgemäßen Verbindung mit der gewünschten Säure. Zweckmäßigerweise wird zur Herstellung von Säureadditionssalzen der Verbindungen der Formel I letztere in einem organischen Lösungsmittel gelöst und mit einer Lösung der gewünschten Säure versetzt. So können beispielsweise die Hydrochloride der erfindungsgemäßen Pyridazinone der Formel I dadurch erhalten werden, daß die Verbindungen in Alkohol gelöst werden und die alkoholische Lösung mit einer äquivalenten Menge einer Lösung von Chlorwasserstoff in Äther versetzt wird.

Die erfindungsgemäßen Tetrahydropyridazinonderivate der Formel I, worin R ein gemäß obiger Definition substituierter Phenylrest oder ein unsubstituierter oder substituierter heterocyclischer Rest ist, sowie auch Tetrahydropyridazinone der Formel I, in denen R ein unsubstituierter Phenylrest ist, und ihre physiologisch verträglichen Salze zeigen ausgeprägte antithrombotische, thrombozytenaggregationshemmende, antianginöse, kardiotone und blutdrucksenkende Wirkungen. Sie sind den bisher bekannten Verbindungen gleicher Wirkungsrichtung überraschenderweise erheblich überlegen und eignen sich daher vorzüglich zur Behandlung von Erkrankungen des Herzens und des Kreislaufsystems. Sie zeigen eine ausgezeichnete Wirksamkeit in verschiedenartigen Tests, wie z.B. Thrombozytenaggregation nach Born, Nature 194, S. 927, (1961); Arachidonsäureletalität am Kaninchen, Science 193, S. 1085, (1974); arterielle und venöse Thromboseverhinderung beim Kaninchen, günstiges hämodynamisches Profil nach per os-Applikation am wachen Hund. Die Prüfung in den genannten und einer Reihe weiterer Tests ergibt, daß die erfindungsgemäß herstellbaren Verbindungen bei geringer Toxizität überraschenderweise ein besonders günstiges, bei bekannten Präparaten in dieser Form nicht vorliegendes Wirkungsprofil aufweisen.

Die erfindungsgemäßen Tetrahydropyridazinonderivate der Formel I, worin R ein gemäß obiger Definition substituierter Phenylrest oder ein gegebenenfalls substituierter heterocyclischer Rest ist, sowie Tetrahydropyridazinone der Formel I, worin R unsubstituiertes Phenyl ist, können daher am Menschen für sich allein oder in Mischungen untereinander, vorzugsweise in Form pharmazeutischer

Zubereitungen, verabreicht werden, und sind insoweit insgesamt Gegenstand der Erfindung.

Die erfindungsgemäßen pharmazeutischen Zubereitungen enthalten als aktiven Bestandteil eine wirksame Dosis mindestens eines erfindungsgemäßen Tetrahydropyridazinonderivats oder eines Säureadditionssalzes davon.

Die Dosierung der erfindungsgemäßen Tetrahydropyridazinonderivate liegt im allgemeinen zwischen 0,1 und 1000 mg, vorzugsweise 1 und 100 mg, pro Tag und wird in bekannter Weise an die Erfordernisse des Einzelfalls angepaßt.

Je nach Art der Applikation bewegt man sich innerhalb des obigen Dosierungsbereichs, um in bekannter Weise den unterschiedlichen Resorptionsbedingungen Rechung zu tragen.

So wählt man bei intravenöser Applikation eine Dosierung im unteren Teil, bei oraler Applikation eine Dosierung mehr im oberen Teil des angegebenen Dosierungsbereichs. Die tägliche Dosis wird normalerweise in mehreren, beispielsweise 2, Teildosen verabreicht, wobei die Einzeldosis in der Regel bei 0,01 bis 10 mg pro kg Körpergewicht liegt.

Die pharmazeutischen Zubereitungen enthalten in der Regel 0,05 bis 100 mg, vorzugsweise 1 bis 50, mg pro Einheit eines oder mehrerer erfindungsgemäßer Tetrahydropyridazinonderivate oder deren pharmakologisch akzeptabler Salze neben üblichen, pharmazeutisch einwandfreien Träger- oder Zusatzstoffen.

Geeignete Trägerstoffe sind z.B. Wasser, pflanzliche öle, Stärke, Gelatine, Milchzucker, Magnesiumstearat, Wachse, Vaseline etc.. Als Zusatzstoffe können z.B. Netzmittel, Sprengmittel, Konservierungsmittel etc. verwendet werden.

Die pharmazeutischen Präparate können in Form von z.B. Tabletten, Kapseln, wäßrigen oder öligen Lösungen oder Suspensionen, Emulsionen, injizierbaren wäßrigen oder öligen Lösungen oder Suspensionen, dispergierbaren Pulvern oder Aerosolmischungen vorliegen. Die pharmazeutischen Präparate können neben den Verbindungen der allgemeinen Formel I auch noch eine oder mehrere andere pharmazeutisch wirksame Substanzen, beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen wie Digoxin, Acetyldigoxin, Metildigoxin und Lanato-Glykoside; Coronardilatatoren, wie Carbocromen, Dipyridamol, Nifedipin und Perhexilin; antianginöse Verbindungen wie Isosorbiddinitrat, Isosorbidmononitrat, Glyceroltrinitrat, Molsidomin und Verapamil; ß-Blocker wie Propranolol, Oxprenolol, Atenolol, Metoprolol und Penbutolol und oogenmetabolische Mittel, wie Pirilinol, enthalten.

Die folgenden Ausführungsbeispiele veranschaulichen die Erfindung. Die Reaktionsbedingungen, die Reduktions-, Alkylierungs- und Acylierungsmittel können im Rahmen des Standes der Technik und der Patentansprüche und die eingesetzten Tetrahydropyridazinonderivate im Rahmen der Patentansprüche variiert werden.

Beispiel 1

6-(3,4-Dimethoxyphenyl)-5-hydroxymethyl-2,3,4,5-tetrahydro-pyridazin-3-on

15,0 g (0,06 mol) 4-(3,4-Dimethoxyphenyl)-carbonyl- -butyrolacton und 3,5 g (0,07 mol) Hydrazinhydrat werden in 200 ml Ethanol 10 h unter Rückfluß erhitzt. Nach Abkühlen wird das ausgefallene Produkt abgesaugt, gewaschen und getrocknet.

Ausbeute: 11,6 g, Schmelzpunkt: 203 - 205°C
Elementaranalyse: ($C_{13}H_{16}N_2O_4$)
ber. (%): C 59,1 H 6,1 N 10,6 O 24,2
gef. (%): C 59,0 H 6,1 N 10,5 O 24,3

Das oben als Ausgangsmaterial eingesetzte 4-(3,4-Dimethoxyphenyl)-carbonyl-$\gamma$-butyrolacton kann wie folgt hergestellt werden:
23,8 g (0,1 mol) 4-(3,4-Dimethoxyphenyl)-4-oxo-buttersäure werden in 220 ml 2%iger Natronlauge gelöst, dann werden 8,5 g 39%ige Formaldehydlösung zugesetzt und 3h bei 50°C gerührt. Nach Ansäuern mit Salzsäure wird über Nacht bei Raumtemperatur gerührt, das halbfeste Produkt abgesaugt, in Essigester aufgenommen, mit Natriumbicarbonat gewaschen, getrocknet und eingeengt.

Ausbeute: 5,6 g Schmelzpunkt: 114 - 116°C
Elementaranalyse:
ber. (%): C 62,4 H 5,6 O 32,0
gef. (%): C 63,2 H 5,7 O 30,9

Die erforderliche 4-(3,4-Dimethoxyphenyl)-4-oxo-buttersäure wird wie folgt erhalten:

240 g Aluminiumchlorid, wasserfrei werden in 800 ml Nitrobenzol gelöst und unter Rühren und Kühlen 88 g (0,88 mol) Bernsteinsäureanhydrid und 128 g (0,93 mol) Veratrol zugesetzt. Nach 24 h Reaktionszeit bei Raumtemperatur wird auf ein Gemisch aus Eis, Wasser, und konz. Salzsäure gegossen und das Nitrobenzol durch Wasserdampfdestillation entfernt, abgekühlt und der ausgefallene Niederschlag abgesaugt.

Zur Reinigung wird in Sodalösung in der Hitze gelöst, über Kohle filtriert, angesäuert, abgesaugt, neutral gewaschen und getrocknet.

Ausbeute: 137 g    Schmelzpunkt 156 - 158°C

Elementaranalyse: ($C_{12}H_{14}O_5$)

ber. (%): C 60,5 H 5,9 O 33,6

gef. (%): C 60,5 H 5,8 O 33,4

Beispiel 2

6-(4-Methylphenyl)-5-hydroxymethyl-2,3,4,5-tetrahydro-pyridazin-3-on

10,2 g (0,05 mol) 4-(4-Methylphenyl)-carbonyl-γ-butyrolacton und 3,0 ml (0,06 mol) Hydrazinhydrat werden in 150 ml Ethanol 1 h unter Rückfluß erhitzt. Die erhaltene Lösung wird im Vakuum eingedampft und der Rückstand in Methylenchlorid aufgenommen. Die Methylenchloridlösung wird erforderlichenfalls filtriert und dann mit dem gleichen Volumen Wasser kräftig verrührt. Hierbei fällt das gewünschte Produkt aus. Es wird abgesaugt, mit Wasser gewaschen und getrocknet.

Ausbeute: 7,6 g (70 % der Theorie)

Schmelzpunkt 177 - 178°C

Elementatanalyse: ($C_{12}H_{14}N_2O_2$)

ber. (%): C 66.0 H 6.5 N 12.8 O 14.7

gef. (%): C 65.6 H 6.5 N 13.0 O 15.1

Das in obigem Beispiel eingesetzte **4-(4-Methylphenyl)-carbonyl-γ-butyrolacton** wird wie folgt hergestellt:
96,1 g (0,5 mol) 4-(4-Methylphenyl)-4-oxo-buttersäure werden in 1100 ml 0,5 N-Natronlauge gelöst, dann werden 44 ml (0,55 mol) 39%ige Formaldehydlösung zugetropft und 8 h bei Raumtemperatur gerührt. Nach dem Ansäuern mit 55 ml konz. Salzsäure wird 12 h weitergerührt, mit Äther extrahiert, die Ätherphase abgetrennt, mit Natriumhydrogencarbonatlösung gewaschen und eingeengt. Der Rückstand wird aus Essigsäureethylester umkristallisiert.

Ausbeute: 16,1 g,
Schmelzpunkt: 88 - 89°C
Elementaranalyse: ($C_{12}H_{12}O_3$)
ber. (%): C 70,6 H 5,9 O 23,5
gef. (%): C 70,4 H 6,0 O 23,6

Beispiel 3

6-(4-Chlorphenyl)-5-hydroxymethyl-2,3,4,5-tetrahydro-pyridazin-3-on

9,0 g (0,04 mol) 4-(4-chlorphenyl)-carbonyl-γ-butyrolacton und 3 ml (0,06 mol) Hydrazinhydrat werden in 150 ml Ethanol 1 h unter Rückfluß erhitzt. Die erhaltene Lösung wird zur Trockene eingedampft, und der Rückstand wird aus Methanol umkristallisiert.

Ausbeute: 3,1 g 32 % der Theorie
Schelzpunkt: 170 - 172°C
Elementaranalyse ($C_{11}H_{11}ClN_2O_2$)
ber. (%): C 55,4 H 4,6 N 11,7 O 13,4 Cl 14,9
gef. (%): C 55,0 H 4,9 N 11,3 O 12,9 Cl 14,5

Das in obigem Beispiel eingesetzte 4-(4-Chlorphenyl)-carbonyl-γ-butyrolacton wird wie folgt hergestellt:
106,3 g (0,5 mol) 4-(4-Chlorphenyl)-4-oxo-buttersäure, 1100 ml 0,5 N-Natronlauge und 44 ml (0,55 mol) 39%ige Formaldehydlösung werden, wie oben beschrieben, umgesetzt und aufgearbeitet.

Ausbeute: 47,6 g
Schmelzpunkt: 82 - 83°C
Elementaranalyse: ($C_{11}H_9ClO_3$)
ber.(%): C 58,8 H 4,0 Cl 15.8 O 21,4
gef.(%): C 59,1 H 4,0 Cl 15,3 O 21,8

Beispiel 4

6-(4-Acetylaminophenyl)-5-hydroxymethyl-2,3,4,5-tetrahydro-pyridazin-3-on

9,9 g (0,04 mol) 4-(4-Acetylaminophenyl)-carbonyl-$\gamma$-butyrolacton und 2,5 g (0,05 mol) Hydrazinhydrat werden in 150 ml Ethanol 2 h unter Rückfluß erhitzt und, wie im Beispiel 3 beschrieben, aufgearbeitet.

Ausbeute: 3,6 g (34% der Theorie)
Schmelzpunkt: 229 - 230°C
Elementaranalyse: ($C_{13}H_{15}N_3O_3$)

| | | | | | | | | |
|---|---|---|---|---|---|---|---|---|
| ber.(%): | C | 59,8 | H | 5,8 | N | 16,1 | O | 18,4 |
| gef.(%): | C | 59,6 | H | 5,8 | N | 15,6 | O | 18,9 |

Das im obigen Ausführungsbeispiel eingesetzte 4-(4-Acetyl-aminophenyl)-carbonyl-$\gamma$-butyrolacton wird wie folgt hergestellt:

94,1 g (0,4 mol) 4-(4-Acetylaminophenyl)-4-oxo-buttersäure, 880 ml 0,5 N Natronlauge und 35 ml (0,44 mol), 39%ige Formaldehydlösung werden, wie oben beschrieben, eingesetzt und aufgearbeitet.

Ausbeute: 27 g
Schmelzpunkt: 152 - 154°C
Elementaranalyse: ($C_{13}H_{13}NO_4$)

| | | | | | | | | |
|---|---|---|---|---|---|---|---|---|
| ber.(%): | C | 63,2 | H | 5,3 | O | 25,9 | N | 5,7 |
| gef.(%): | C | 63,1 | H | 5,5 | O | 26,5 | N | 5,3 |

Beispiel 5

Pyridin-3-carbonsäure-/(6-(3,4-dimethoxyphenyl)-3-oxo-2,3,4,5-tetrahydro-pyridazin-5-yl)-methyl/-ester

1,3 g (0,01 mol) Nicotinsäure und 1,7 g (0,01 mol) Carbonyldiimidazol werden in 30 ml Tetrahydrofuran 15 Minuten unter Rückfluß erhitzt. Nach dem Abkühlen werden 2,6 g (0,01 mol) 6-(3,4-Dimethoxyphenyl)-5-hydroxymethyl-2,3,4,5-tetrahydro-pyridazin-3-on unter Rühren zugegeben. Nach 12 Stunden Reaktionszeit wird eingedampft, der Rückstand in Methylenchlorid aufgenommen, mit Natriumhydrogencarbonatlösung gewaschen, getrocknet, eingedampft und der Rückstand aus Methanol umkristallisiert.

Ausbeute: 2,5 g (68 % der Theorie)
Schmelzpunkt: 89 - 91°C
Elementaranalyse: ($C_{19}H_{19}N_3O_5$)

| | | | | | | | | |
|---|---|---|---|---|---|---|---|---|
| ber.(%): | C | 61,8 | H | 5,2 | N | 11,4 | O | 21,7 |
| gef.(%): | C | 60,6 | H | 5,9 | N | 10,9 | O | 22,4 |

In analoger Weise lassen sich beispielsweise herstellen:

6-(4-Dimethylaminophenyl)-5-hydroxymethyl-2,3,4,5-tetrahydro-pyridazin-3-on

Essigsäure-((6-(3,4-dimethoxyphenyl)-3-oxo-2,3,4,5-tetrahydro-pyridazin-5-yl)-methyl)-ester,

Pyridin-3-carbonsäure-((6-(4-chlorphenyl)-3-oxo-2,3,4,5-tetrahydro-pyridazin-5-yl)-methyl)-ester,

2-(2-Dimethylaminoethyl)-5-hydroxymethyl-6-(4-methylphenyl)-2,3,4,5-tetrahydro-pyridazin-3-on,

6-(4-Acetoxyphenyl)-5-hydroxymethyl-2,3,4-5-tetrahydropyridazin-3-on,

6-(4-Bromphenyl)-5-hydroxymethyl-2,3,4,5-tetrahydropyridazin-3-on,

6-(3-Aminophenyl)-5-hydroxymethyl-2,3,4,5-tetrahydropyridazin-3-on,

5-Hydroxymethyl-6-(3-hydroxyphenyl)-2,3,4,5-tetrahydro-pyridazin-3-on,

6-(3,4-Dimethoxyphenyl)-5-hydroxymethyl-2-phenyl-2,3,4,5-tetrahydro-pyridazin-3-on,

6-(3-Methoxyphenyl)-5-hydroxymethyl-2,3,4,5-tetrahydro-pyridazin-3-on,

5-Hydroxymethyl-6-(2-methylphenyl)-2,3,4,5-tetrahydro-pyridazin-3-on,

5-Hydroxymethyl-6-(3-pyridyl)-2,3,4,5-tetrahydro-pyridazin-3-on,

6-(2-Chlorphenyl)-5-hydroxymethyl-2,3,4,5-tetrahydro-pyridazin-3-on,

5-Hydroxymethyl-6-(3,4,5-trimethoxyphenyl)-2,3,4,5-tetra-hydro-pyridazin-3-on.

## Beispiel 6

### 4-Chlorphenoxyessigsäure-(6-(3,4-dimethoxyphenyl)-3-oxo-2,3,4,5,-tetrahydro-pyridazin-5-yl)-methyl-ester

4,7 g (0,025 mol) 4-Chlorphenoxyessigsäure und 4,3 g (0,027 mol) Carbonyldiimidazol werden in 100 ml Tetrahydrofuran 15 min unter Rückfluß erhitzt. Nach dem Abkühlen werden 6,7 g (0,025 mol) 6-(3,4-Dimethoxyphenyl)-5-hydroxymethyl-2,3,4,5-tetrahydropyridazin-3-on unter Rühren zugegeben. Nach 5 Stunden Reaktionszeit wird bei Raumtemperatur eingeengt und wie in Beispiel 5 aufgearbeitet.

Ausbeute: 8,1 g (75% der Theorie)
Schmelzpunkt: 150 - 151 °C
Elementaranalyse: $C_{21}H_{21}ClN_2O_6$ (432.86)

ber: (%) C 58,3 H 4,9 Cl 8,2 N 6,5 O 22,2
gef: (%) C 58,3 H 4,8 Cl 8,3 N 6,3 O 22,1

Beispiel 7

6-(4-Acetylaminophenyl)-5-hydroxymethyl-2-methyl-2,3,4,5-tetrahydropyridazin-3-on

7,4 g (0,03 mol) 4-(4-Acetylaminophenyl)-carbonyl-γ-butyrolacton und 2 ml (0,038 mol) Methylhydrazin werden in 30 ml Ethanol 1 Stunde bei Raumtemperatur, dann 3 Stunden unter Rückfluß erhitzt. Nach dem Abkühlen wird das ausgefallene Produkt abgesaugt und gewaschen.

Ausbeute: 4,6 g (56% der Theorie)
Schmelzpunkt: 219 - 221°C
Elementaranalyse: $C_{14}H_{17}N_3O_3$ (275.31)

ber: (%) C 61,1 H 6,2 N 15,3 O 17,4
gef: (%) C 61,0 H 5,9 N 15,4 O 17,7

Beispiel 8

6-(4-Acetylaminophenyl)-5-hydroxymethyl-2-phenyl-2,3,4,5-tetrahydropyridazin-3-on

4,7 g (0,019 mol) 4-(4-Acetylaminophenyl)-carbonyl-γ-butyrolacton und 2 ml (0,02 mol) Phenylhydrazin werden in 30 ml Ethanol 1 Stunde bei Raumtemperatur, dann 16 Stunden unter Rückfluß gerührt. Das ausgefallene Produkt wird abgesaugt und aus 90% Ethanol umkristallisiert.

Ausbeute: 1,0 g (16% der Theorie)
Schmelzpunkt: 221-222°C
Elementaranalyse: $C_{19}H_{19}N_3O_3$ (337.38)

ber: (%) C 67,6 H 5,7 N 12,5 O 14,2

gef: (%) C 67,5 H 5,9 N 12,6 O 14,1

Beispiel 9:

2,3,4,5-Tetrahydro-6-(2-furyl)-5-hydroxymethyl-pyridazin-3-on

4,7 g (0,026 mol) 4-(2-Furyl)-carbonyl-γ-butyrolacton und 1,5 ml (0,03 mol) Hydrazinhydrat werden in 40 ml Ethanol bei Raumtemperatur gelöst und 1 Stunde bei 50°C gerührt. Nach dem Einengen wird das Reaktionsprodukt über eine Kieselgelsäure unter Verwendung von Methylenchlorid/Methanol = 9:1 als Laufmittel fraktioniert chromatographiert.

Ausbeute: 1,4 g (28 % d. Theorie)

Schmelzpunkt: 161 - 163°C

Elementaranalyse: $C_9H_{10}N_2O_3$ (194,19)

berechnet: C 55,7 H 5,2 N 14,4 O 24,7

gefunden: C 55,6 H 4,9 N 14,4 O 25,0

Beispiel 10:

a) 4-(2-Pyrrolyl)-carbonyl-γ-butyrolacton-hydrazon-hydrat

7,2 g (0,04 mol) 4-(2-Pyrrolyl)-carbonyl-γ-butyrolacton und 2,5 ml (0,05 mol) Hydrazinhydrat werden 4 Stunden bei Raumtemperatur in Ethanol gerührt. Das auskristallisierte Produkt wird abgesaugt und mit Ethanol nachgewaschen.

Ausbeute: 7,0 g (83 % d. Theorie)

Schmelzpunkt: 140 - 142°C

Elementaranalyse: $C_9H_{13}N_3O_3$ (211,22)

berechnet: C 51,2 H 6,2 N 19,9 O 22,7

gefunden: C 51,0 H 6,2 N 20,0 O 23,0

b) 2,3,4,5-Tetrahydro-5-hydroxymethyl-6-(2-pyrrolyl)-pyridazin-3-on

5,2 g (0,025 mol) der im Abschnitt a) hergestellten Verbindung werden mit 50 ml Ethanol, das 1 Tropfen ethanolische Salzsäure enthält, 90 Minuten unter Rückfluß erhitzt. Nach dem Einengen des Ansatzes wird der Rückstand mit Petrolether angerieben, wobei er kristallisiert. Die erhaltenen rohen Kristalle werden abgesaugt und aus Ethanol umkristallisiert.

Ausbeute: 2,2 g (46 % d. Theorie)
Schmelzpunkt: 202 - 203°C
Elementaranalyse: $C_9H_{11}N_3O_2$ (193,21)
berechnet: C 56,0 H 5,7 N 21,7 O 16,6
gefunden: C 56,4 H 5,9 N 21,9 O 16,5

Beispiel 11:

2,3,4,5-Tetrahydro-5-hydroxymethyl-6-(3-nitrophenyl)-pyridazin-3-on

3,5 g (0,015 mol) 4-(3-Nitrophenyl)-carbonyl-γ-butyrolacton und 0,8 ml (0,016 mol) Hydrazinhydrat werden 30 Minuten bei Raumtemperatur und danach 60 Stunden unter Rückfluß erhitzt. Nach dem Einengen wird der Rückstand aus Essigsäureethylester/Petrolether fraktioniert kristallisiert.

Ausbeute: 1,1 g (30 % d. Theorie)
Schmelzpunkt: 246 - 248°C
Elementaranalyse: $C_{11}H_{11}N_3O_4$ (249,23)
berechnet: C 53,0 H 4,4 N 16,9 O 25,7
gefunden: C 53,0 H 4,6 N 16,8 O 26,0

Beispiel 12.

2,3,4,5-Tetrahydro-5-hydroxymethyl-6-(3-indolyl)-pyridazin-3-on

3,3 g (0,014 mol) 4-(3-Indolyl)-carbonyl-γ-butyrolacton und 0,85 ml (0,018 mol) Hydrazinhydrat werden in 50 ml Ethylenglykolmonomethylether und 1 ml Essigsäure 18 Stunden bei 80°C gerührt. Nach Zugabe von 10 ml Petrolether zum Reaktionsgemisch kristallisiert das Produkt in guter Reinheit aus.

Ausbeute: 1,2 g (35 % d. Theorie)
Schmelzpunkt: 235 - 237°C

Elementaranalyse: $C_{13}H_{13}N_3O_2$ (243,27)

| | | | | |
|---|---|---|---|---|
| berechnet: | C 64,2 | H 5,4 | N 17,3 | O 13,2 |
| gefunden: | C 63,8 | H 5,3 | N 17,1 | O 13,5 |

Beispiel 13:

2,3,4,5-Tetrahydro-5-hydroxymethyl-6-(4-(2-(1-imidazolyl)-ethoxy)-phenyl)-pyridazin-3-on

5,2 g (0,017 mol) 4-(4-(2-(1-Imidazolyl)-ethoxy)-phenyl)-carbonyl-γ-butyrolacton und 1 ml (0,02 mol) Hydrazinhydrat werden in 30 ml Ethanol 1 Stunde bei Raumtemperatur, dann 16 Stunden bei 60°C gerührt. Nach dem Abkühlen wird der Ansatz mit 15 ml Ether versetzt, das ausgefallene Produkt abgesaugt, mit Ether gewaschen und getrocknet.

Ausbeute: 2,7 g (51 % d. Theorie)
Schmelzpunkt: 199 - 200°C
Elementaranalyse: $C_{16}H_{18}N_4O_3$ (314,35)

| | | | | |
|---|---|---|---|---|
| berechnet: | C 61,1 | H 5,8 | N 17,8 | O 15,3 |
| gefunden: | C 61,5 | H 5,6 | N 18,1 | O 15,1 |

**Beispiel 14:**

**6-(4-Methoxyphenyl)-5-hydroxymethyl-2,3,4,5-tetrahydro-pyridazin-3-on**

9,4 g (0,043 mol) 4-(4-Methoxyphenyl-carbonyl)-γ-butyrolacton und 4 g (0,08 mol) Hydrazinhydrat werden in 80 ml Ethanol 6 Stunden unter Rückfluß erhitzt. Nach dem Einengen wird der Rückstand aus Wasser umkristallisiert.

Ausbeute: 2,5 g (25 % d. Theorie)
Schmelzpunkt: 154 - 156°C
Elementaranalyse: $C_{12}H_{14}N_2O_3$ (234,26)
berechnet: C 61,5 H 6,0 N 12,0 O 20,5
gefunden: C 62,0 H 6,1 N 11,6 O 20,5

**Beispiel 15:**

**6-(2-Thienyl)-5-hydroxymethyl-2,3,4,5-tetrahydro-pyridazin-3-on**

11,8 g (0,06 mol) 4-(2-Thienyl-carbonyl)-γ-butyrolacton und 3,1 g (0,062 mol) Hydrazinhydrat werden in 100 ml Ethanol 2 Stunden bei Raumtemperatur gerührt. Das ausgefallene Hydrazon wird abgesaugt, mit 60 ml Toluol und 6 ml Essigsäure versetzt und 2 Stunden unter Rückfluß erhitzt. Nach Abkühlen wird das ausgefallene Produkt abgesaugt, gewaschen und getrocknet.

Ausbeute: 3,6 g (29 % d. Theorie)
Schmelzpunkt: 160 - 162°C
Elementaranalyse: $C_9H_{10}N_2O_2S$ (210,19)
berechnet: C 51,4 H 4,8 N 13,3 O 15,2 S 15,2
gefunden: C 50,9 H 4,8 N 13,4 O 15,4 S 15,2

Beispiel 16:

6-(4-(5-Methyl-1,3,4-oxdiazol-2-yl)-methoxyphenyl)-5-hydroxymethyl-2,3,4,5-tetrahydro-pyridazin-3-on

9,0 g (0,03 mol) 4-(4-(5-Methyl-1,3,4-oxdiazol-2-yl)-methoxy-phenyl-carbonyl)-γ-butyrolacton und 1,6 g (0,032 mol) Hydrazinhydrat werden in 50 ml Ethanol 12 Stunden bei Raumtemperatur gerührt. Nach Zugabe von 5 ml Essigsäure wird 2 Stunden unter Rückfluß erhitzt, eingeengt, in Essigsäureethylester gelöst und mit wäßriger Kaliumcarbonatlösung extrahiert. Die organische Phase wird eingeengt und der Rückstand mit Methylenchlorid kristallisiert.

Ausbeute: 6 g (64 % d. Theorie)
Schmelzpunkt: 134 - 136°C
Elementaranalyse: $C_{15}H_{16}N_4O_4$ (316,32)

| | | | | |
|---|---|---|---|---|
| berechnet: | C 57,0 | H 5,1 | N 17,7 | O 20,2 |
| gefunden: | C 56,7 | H 5,3 | N 17,2 | O 21,3 |

Beispiel 17:

6-(4-(1-Pyrrolidinyl-2-on)-phenyl)-5-hydroxymethyl-2,3,-4,5-tetrahydro-pyridazin-3-on

8,4 g (0,031 mol) 4-(4-(1-Pyrrolidinyl-2-on)-phenyl-carbonyl)-γ-butyrolacton und 1,7 g (0,034 mol) Hydrazinhydrat werden in 50 ml Ethanol 2 Stunden bei 50°C gerührt, abgekühlt und abgesaugt. Das erhaltene Hydrazon wird mit 70 ml Ethanol und 5 ml Essigsäure 2 Stunden unter Rückfluß erhitzt, abgekühlt, abgesaugt und getrocknet.

Ausbeute: 4,2 g (48 % d. Theorie)
Schmelzpunkt: 219 - 220°C
Elementaranalyse: $C_{15}H_{17}N_3O_3$ (287,32)

berechnet: C 62,7 H 6,0 N 14,6 O 16,7
gefunden: C 62,1 H 6,4 N 14,5 O 17,5

Beispiel 18:

6-(4-(2-Methoxyethoxy)-phenyl)-5-hydroxymethyl-2,3,4,5-tetrahydro-pyridazin-3-on

13,7 g (0,052 mol) 4-(4-(2-Methoxyethoxy)-phenyl-carbonyl)-γ-butyrolacton werden mit 2,8 g (0,056 mol) Hydrazinhydrat in 80 ml Ethanol, wie vorstehend beschrieben, umgesetzt.

Ausbeute: 12,5 g (87 % d. Theorie)
Schmelzpunkt: 187 - 188°C
Elementaranalyse: $C_{14}H_{18}N_2O_4$ (278,31)
berechnet: C 60,4 H 6,5 N 10,1 O 23,0
gefunden: C 60,9 H 7,0 N 10,2 O 21,8

Beispiel 19:

6-(4-(3-Pyridyl-methoxy)-phenyl)-5-hydroxymethyl-2,3,4,5-tetrahydro-pyridazin-3-on

7,5 g (0,025 mol) 4-(4-(3-Pyridyl-methoxy)-phenyl-carbonyl)-γ-butyrolacton werden mit 1,4 g (0,028 mol) Hydrazinhydrat in 50 ml Ethanol, wie vorstehend beschrieben, umgesetzt.

Ausbeute: 6,4 g (82 % d. Theorie)
Schmelzpunkt: 222 - 223°C
Elementaranalyse: $C_{17}H_{17}N_3O_3$ (311,34)
berechnet: C 65,6 H 5,5 N 13,5 O 15,4
gefunden: C 65,8 H 5,4 N 13,8 O 15,1

Beispiel 20:

6-(4-(4-Methyl-2-pyridon-6-yl)-methoxy-phenyl)-5-hydroxymethyl-2,3,4,5-tetrahydro-pyridazin-3-on

6 g (0,018 mol) 4-((4-Methyl-2-pyridon-6-yl)-methoxyphenyl)-carbonyl-γ-butyrolacton und 1 g (0,02 mol) Hydrazinhydrat werden in 50 ml Ethanol, wie vorstehend beschrieben, umgesetzt.

Ausbeute: 5,4 g (88 % d. Theorie)
Schmelzpunkt: 65 - 68°C
Elementaranalyse: $C_{18}H_{19}N_3O_4$ (341,37)

| | | | | |
|---|---|---|---|---|
| berechnet: | C 63,3 | H 5,6 | N 12,3 | O 18,7 |
| gefunden: | C 62,8 | H 5,3 | N 12,5 | O 19,2 |

Beispiel 21:

6-(4-Methylmercaptophenyl)-5-hydroxymethyl-2,3,4,5-tetrahydropyridazin-3-on

10,6 g (0,045 mol) 4-(4-Methylmercaptophenyl)-carbonyl-γ-butyrolacton werden in 70 ml Ethanol mit 2,5 g (0,05 mol) Hydrazinhydrat 90 Minuten bei Raumtemperatur gerührt. Nach Zugabe von 8 ml Essigsäure wird 1 Stunde unter Rückfluß erhitzt, abgekühlt, abgesaugt und getrocknet.

Ausbeute: 7,1 g (63 % d. Theorie)
Schmelzpunkt: 158-160°C
Elementaranalyse: ($C_{12}H_{14}N_2O_2S$) (250,32)

| | | | | | |
|---|---|---|---|---|---|
| berechnet: | C 57,6 | H 5,6 | N 11,2 | O 12,8 | S 12,8 |
| gefunden: | C 57,8 | H 5,6 | N 11,1 | O 12,9 | S 12,7 |

Beispiel 22:

6-(4-(2-Methoxyethyl-aminocarbonylmethoxy)-phenyl)-5-hydroxymethyl-2,3,4,5-tetrahydropyridazin-3-on

4,0 g (0,012 mol) 4-(4-(2-Methoxyethylamino-carbonylmethoxy)-phenyl)-carbonyl-γ-butyrolacton und 0,7 g (0,014 mol) Hydrazinhydrat werden in 20 ml Ethanol, wie vorstehend beschrieben, umgesetzt.

Ausbeute: 2,1 g (52 % d. Theorie)
Schmelzpunkt: 169-171°C
Elementaranalyse: $(C_{16}H_{21}N_3O_5)$ (335,36)
berechnet: C 57,3 H 6,3 N 12,5 O 23,9
gefunden: C 57,0 H 6,0 N 12,4 O 24,5

Beispiel 23:

6-(4-(Aminocarbonylmethoxy)-phenyl)-5-hydroxymethyl-2,3,4,5-tetrahydropyridazin-3-on

11,3 g (0,043 mol) 4-(4-(Aminocarbonylmethoy)-phenyl)-carbonyl-γ-butyrolacton und 2,4 g (0,048 mol) Hydrazinhydrat werden in 70 ml Ethanol, wie vorstehend beschrieben, umgesetzt.

Ausbeute: 6,5 g (55 % d. Theorie)
Schmelzpunkt: 238-240°C
Elementaranalyse: $(C_{13}H_{15}N_3O_4)$ (277,28)
berechnet: C 56,3 H 5,5 N 15,2 O 23,1
gefunden: C 55,5 H 5,9 N 15,6 O 24,0

Beispiel 24:

6-(3,4-Dimethoxyphenyl)-5-hydroxymethyl-2-methyl-2,3,4,5-tetrahydropyridazin-3-on

7,5 g (0,03 mol) 4-(3,4-Dimethoxyphenyl)-carbonyl-γ-butyrolacton und 2 ml (0,03 mol) Methylhydrazin werden in 50 ml Ethanol 1 Stunde bei Raumtemperatur und dann 17 Stunden bei 90°C gerührt. Nach dem Abkühlen wird das ausgefallene Produkt abgesaugt und getrocknet.

Ausbeute: 4,3 g (52 % d. Theorie)
Schmelzpunkt: 158-160°C
Elementaranalyse: ($C_{14}H_{18}N_2O_4$) (278,31)
berechnet: C 60,4 H 6,5 N 10,1 O 23,0
gefunden: C 60,2 H 6,6 N 10,2 O 23,0

Beispiel 25:

6-(4-Cyanophenyl)-5-hydroxymethyl-2,3,4,5-tetrahydropyridazin-3-on

3,0 g (0,014 mol) 4-(4-Cyanophenyl)-carbonyl-γ-butyrolacton und 1 ml (0,021 mol) Hydrazinhydrat werden in 50 ml Ethanol und 50 ml DMF 10 Stunden bei Raumtemperatur gerührt. Nach Zugabe von 1 ml Essigsäure wird 18 Stunden bei 80°C gerührt und wie oben aufgearbeitet.
Das Produkt wird durch Zugabe von je 2 ml Diethylether, Petrolether und Essigsäureethylester kristallisiert.

Ausbeute: 1,2 g (38 % d. Theorie)
Schmelzpunkt: 183-186°C
Elementaranalyse: ($C_{12}H_{11}N_3O_2$) (229,24)
berechnet: C 62,9 H 4,8 N 18,3 O 14,0
gefunden: C 61,9 H 4,9 N 17,7 O 14,2

Beispiel 26:

6-((4-Hydroxycarbonylmethoxy)-phenyl)-5-hydroxymethyl-2,3,4,5-tetrahydropyridin-3-on

6,5 g (0,025 mol) 4-(4-(Hydroxycarbonylmethoxy)-phenyl)-carbonyl-γ-butyrolacton und 2,8 g (0,056 mol) Hydrazinhydrat werden in 50 ml Ethanol 3 Stunden bei Raumtemperatur gerührt. Nach Zugabe von 8 ml Essigsäure wird 1 Stunde unter Rückfluß erhitzt, abgekühlt, abgesaugt und getrocknet.

Ausbeute: 6,8 g (99 % d. Theorie)
Schmelzpunkt: 187°C
Elementaranalyse: ($C_{13}H_{14}N_2O_5$) (278,27)
berechnet: C 56,1 H 5,1 N 10,1 O 28,7
gefunden: C 56,0 H 5,2 N 10,2 O 28,9

Beispiel 27:

a) 4-(3,4,5-Trimethoxyphenyl)-carbonyl-γ-butyrolacton-hydrazon-hydrat

11,2 g (0,04 mol) 4-(3,4,5-Trimethoxyphenyl)-carbonyl-γ-butyrolacton und 2 ml (0,04 mol) Hydrazinhydrat werden in 60 ml Ethanol 1 Stunde bei Raumtemperatur gerührt und abgesaugt.

Ausbeute: 10,5 g (84 % d. Theorie)
Schmelzpunkt: 154-157°C

b) 2,3,4,5-Tetrahydro-5-hydroxymethyl-6-(3,4,5-trimethoxy-phenyl)-pyridazin-3-on

7,0 g (0,022 mol) der im Abschnitt a) hergestellten Verbindung werden in 20 ml DMF und 1 ml Essigsäure 5 Stunden bei 80°C gerührt. Nach dem Einengen wird in Methylenchlorid aufgenommen, mit Kaliumcarbonatlösung gewaschen, eingeengt und aus Ethanol umkristallisiert.

Ausbeute: 4,0 g (61 % d. Theorie)
Schmelzpunkt: 173-175°C
Elementaranalyse: ($C_{14}H_{18}N_2O_5$) (294,31)
berechnet: C 57,1 H 6,2 N 9,5 O 27,2
gefunden: C 57,0 H 5,8 N 9,7 O 27,9

Beispiel 28:

4-(3-Indolyl)-carbonyl-γ-butyrolacton

Diese als Ausgangsmaterial für das obige Beispiel 12 eingesetzte Verbindung wird wie folgt hergestellt: 21,7 g (0,1 mol) 4-(3-Indolyl)-4-oxo-buttersäure, 8 ml 39%ige Formaldehydlösung und 130 ml 3,5%ige Natronlauge werden 24 Stunden bei 50°C im Autoklaven gerührt. Nach Zugabe von 10 ml konz. Salzsäure wird 12 Stunden bei Raumtemperatur weitergerührt, mit Methylenchlorid extrahiert, mit Natriumhydrogencarbonatlösung gewaschen, eingeengt und aus Toluol umkristallisiert.

Ausbeute: 10,4 g (45 % d. Theorie)
Schmelzpunkt: 188 - 191°C
Elementaranalyse: $C_{13}H_{11}NO_3$ (229,24)
berechnet: C 68,1 H 4,8 N 6,1 O 20,9
gefunden: C 67,7 H 4,9 N 6,3 O 20,8

In analoger Weise lassen sich weitere 4-Aryl-carbonyl-γ-butyrolactone herstellen, beispielsweise:
4-(3-Nitrophenyl)-carbonyl-γ-butyrolacton
4-(1-Acetyl-2-pyrrolidinyl)-carbonyl-γ-butyrolacton
4-(1-Formyl-2-pyrrolidinyl)-carbonyl-γ-butyrolacton
4-(-2-Pyrrolidinyl)-carbonyl-γ-butyrolacton
4-(2-Pyrrolyl)-carbonyl-γ-butyrolacton
4-(2-Furyl)-carbonyl-γ-butyrolacton
4-(4-Cyanophenyl)-carbonyl-γ-butyrolacton
4-(4-Methoxycarbonylphenyl)-carbonyl-γ-butyrolacton

4-(4-Nitrophenyl)-carbonyl-γ-butyrolacton
4-(4-Aminocarbonylaminophenyl)-carbonyl-γ-butyrolacton
4-(4-(2-(1-Imidazolyl)-ethoxy)-phenyl)-carbonyl-γ-butyrolacton
4-(4-(2-Oxo-1,3-oxazolidin-5-yl)-methoxyphenyl)-carbonyl-γ-butyrolacton
4-(4-(1,4-Dioxobutylenimino)-phenyl)-carbonyl-γ-butyrolacton.

Die folgenden Beispiele veranschaulichen die Zusammensetzung von Zubereitungen der erfindungsgemäßen Tetrahydropyridazinonderivate.

Beispiel A

Tabletten

| | pro Tablette |
|---|---|
| Wirkstoff (feingemahlen) | 50 mg |
| Milchzucker (pulverisiert) | 150 mg |
| Maisstärke, weiß | 230 mg |
| Polyvinylpyrrolidon | 15 mg |
| Magnesiumstearat | 5 mg |
| | 450 mg |

Beispiel B

Injektionslösung

| | | |
|---|---|---|
| Wirkstoff | | 4 mg |
| Natriumchlorid | | 0,7 mg |
| Wasser zu Injektionszwecken | ad | 1 ml |

Beispiel C

Rektale Arzneiform

| | | |
|---|---|---|
| Wirkstoff | | 20 mg |
| Suppositoriumsgrundmasse | ad | 2 g |

0129791
Ref. 3302
Dr.Va/St

## Beispiel D

### Emulsionen

| | | |
|---|---|---|
| Wirkstoff | | 60 mg |
| Glycerin, rein | | 0,2 - 2,0 g |
| Polyethylenstearat | | q.s. |
| Neutralöl | | q.s. |
| Geschmackskorrigens | | q.s. |
| Wasser entsalzt | ad | 100 ml |

## Beispiel E

### Wirkstofflösungen

| | | |
|---|---|---|
| Wirkstoff | | 8 mg |
| Polyethylenglykol | | 1,5 mg |
| Glycofurol | ad | 4 ml |

Diluens:

| | |
|---|---|
| Wasser zu Injektionszwecken | 6 ml |

Die erfindungsgemäßen Tetrahydropyridazinonderivate zeigen bereits in geringer Dosierung eine therapeutisch besonders wertvolle Kombination von antithrombotischer, cardiotoner und antianginöser Wirkung bei geringer Blutdrucksenkung.

Die folgenden Tabellen 2 bis 6 zeigen die in verschiedenen in vivo- und in vitro Tests erhaltenen Wirkungsdaten erfindungsgemäßer Verbindungen.

Die in der Tabelle 2 angegebenen Werte des arteriellen Thromboseschutzes wurden an der Ratte nach der Methode von Meng und Seuter (Naunyn-Schmiedeberg's Arch. Pharmacol., 301, 115 (1977)), die des venösen Thromboseschutzes am Kaninchen nach der Methode von Harbauer ("Versuche zur Entwicklung eines standardisierten venösen Thrombosemodells am Kaninchen", 17. Angiologisches Symposium in Kitzbühel, (1982)) ermittelt.

Die in Tabelle 3 angegebenen Werte der Beeinflussung der Arachidonsäurewirkung wurden am narkotisierten Meerschweinchen nach der Methode von Lefort und Vargaftig, (Br. J. Pharmac. 63, 35 (1978)) bestimmt.

Die in den Tabellen 4 bis 6 angegebenen Werte der Inhibierung der Thrombozytenaggregation wurden in vitro nach der Methode von Born (J. Physiol. 162, 67 P (1962)) unter Einsatz von Arachidonsäure, Thrombin oder Adenosindiphosphat als Aggregationsmittel ermittelt.

0129791
Ref. 3275/3302
Dr.Va/St

## Tabelle 2

Beeinflussung der experimentellen Thrombose *in vitro*

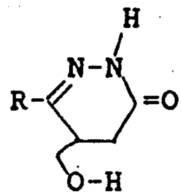

| R | Dosis mg/kg i.p. | Thromboseschutz % arteriell (Ratte) | Thromboseschutz % venös (Kaninchen) |
|---|---|---|---|
| 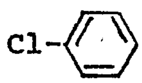 | 10 | | 57 |
| 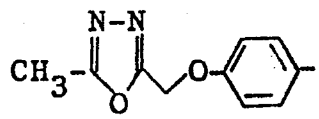 | 10 | | 50 |
| 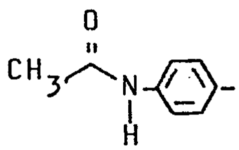 | 0,3<br>1 | 30<br>80 | 60<br>78 |
| 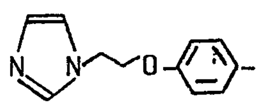 | 3<br>10 | | 57<br>86 |
| 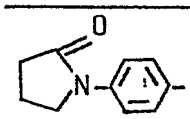 | 10 | | 83 |
| 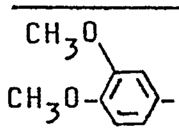 | 0,5<br>1 | 50 | <br>50 |
| 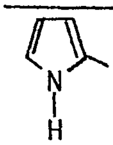 | 1 | 42 | |
| 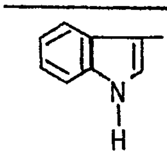 | 1<br>3 | 33 | <br>57 |

Tabelle 3

**Beeinflussung der Arachidonsäurewirkungen (Arachidonsäure 500 µg/kg i.v.) in vitro beim narkotisierten Meerschweinchen**

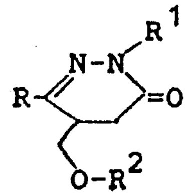

| R | $R^1$ | $R^2$ | Dosis mg/kg i.v. | $TXA_2$-Effekt Broncho-spasmus | Thrombo-zytopenie | $PGI_2$-Effe Blutdruck senkung |
|---|---|---|---|---|---|---|
| 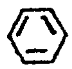 | H | H | 1-10 | (+) | - | (+) |
| 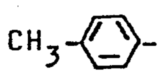 | H | H | 1-10 | (+) | (+) | (+) |
| 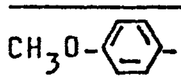 | H | H | 1-10 | (+) | - | ++ |
| 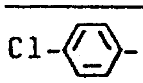 | H | H | 1-10 | (+) | (+) | (+) |
| 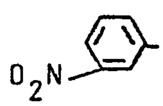 | H | H | 1-10 | ++ | + | (+) |
| 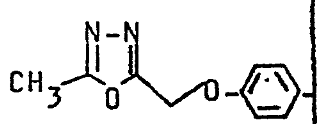 | H | H | 1-10 | ++ | ++ | + |
| 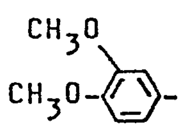 | H | 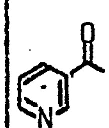 | 1-10 | (+) | (+) | - |
| 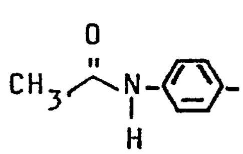 | $CH_3$ | H | 1-10 | (+) | (+) | (+) |
| 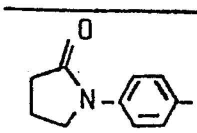 | H | H | 0,3 - 3 | + | ++ | + |

Tabelle 3

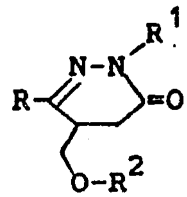

| R | $R^1$ | $R^2$ | Dosis mg/kg i.v. | $TXA_2$ - Effekt Broncho-spasmus | Thrombo-zytopenie | $PGI_2$-Effekt Blutdruck-senkung |
|---|---|---|---|---|---|---|
| 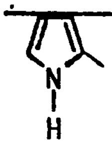 | H | H | 1 - 10 | ++ | +++ | (+) |
| 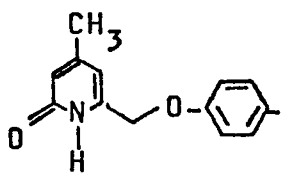 | H | H | 1 - 10 | (+) | (+) | - |
| 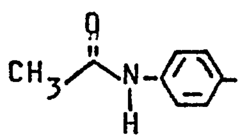 | H | H | o,1 - 1 | ++ | ++ | ++ |
| 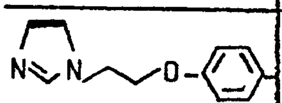 | H | H | 1 - 10 | + | ++ | (+) |
| 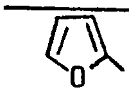 | H | H | 1 - 10 | (+) | (+) | - |
| 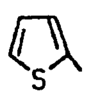 | H | H | 3 - 10 | (+) | (+) | (+) |
| 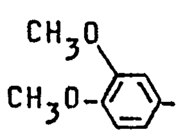 | H | H | 0,3 - 3 | ++ | ++ | +++ |
| 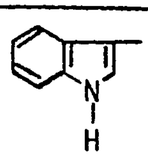 | H | H | 1 - 10 | + | +++ | (+) |

Tabelle 3

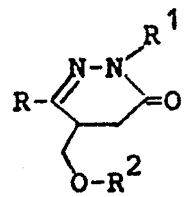

| R | $R^1$ | $R^2$ | Dosis mg/kg i.v. | $TXA_2$-Effekt Bronchospasmus | $TXA_2$-Effekt Thrombozytopenie | $PGI_2$-Effekt Blutdrucksenkung |
|---|---|---|---|---|---|---|
| 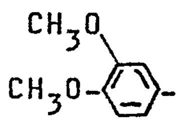 | H | 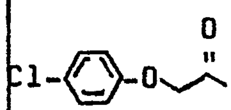 | 1 - 10 | (+) | - | + |
| 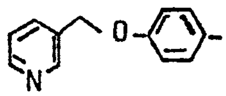 | H | H | 1 - 10 | + | + | - |

- = keine Wirkung
(+) = schwach
+ = mittel
++ = stark
+++ = sehr stark

<u>Tabelle 4</u>

Hemmung der Thrombozytenaggregation <u>in vitro</u> (nach BORN) durch 0,36 mM Arachidonsäure induziert

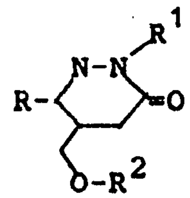

| R | $R^1$ | $R^2$ | $IC_{50}$ (µM) |
|---|---|---|---|
| 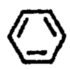 | H | H | 5 |
| 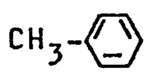 | H | H | 4 |
| 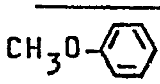 | H | H | 1,5 |
| 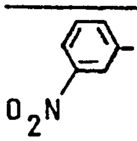 | H | H | 1,5 |
| 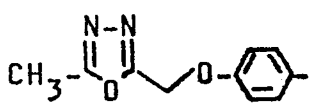 | H | H | 1 |
| 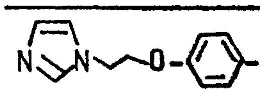 | H | H | 2 |
| 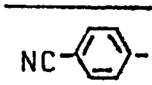 | H | H | 0,3 |
| 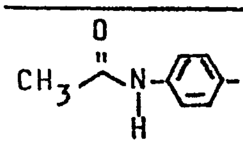 | $CH_3$ | H | 〉100 |
| 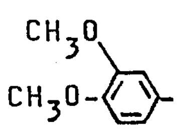 | H | 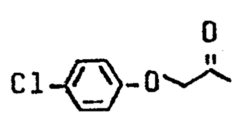 | 4 |
| 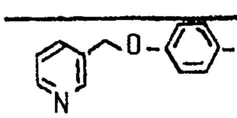 | H | H | 0,45 |

Tabelle 4

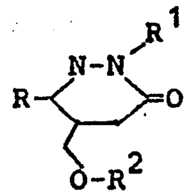

| R | $R^1$ | $R^2$ | $IC_{50}$ (μM) |
|---|---|---|---|
| 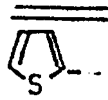 | H | H | 50 |
| 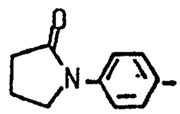 | H | H | 0,4 |
| 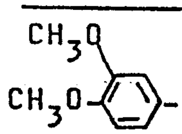 | H | H | 0,6 |
| 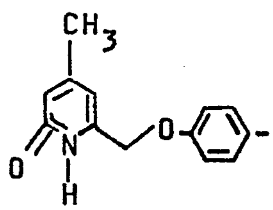 | $CH_3$ | H | 15 |
| 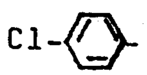 | H | H | 0,9 |
| 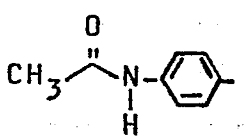 | H | H | 0,2 |
| 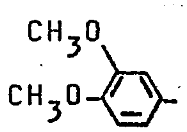 | $CH_3$ | H | 40 |
| 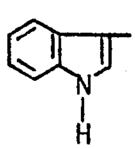 | H | H | 0,55 |

Tabelle 4

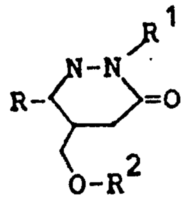

| R | $R^1$ | $R^2$ | $IC_{50}$ (µM) |
|---|---|---|---|
| 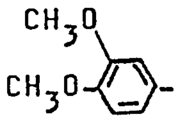 | H | 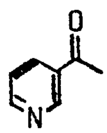 | 30 |
| 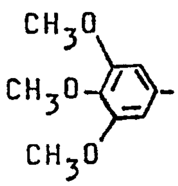 | H | H | 6 |

Tabelle 5

Hemmung der Thrombozytenaggregation in vitro (nach BORN) durch 0,2 - 0,4 N.I.H.-Einheiten/ml induziert

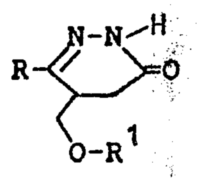

| R | $R^1$ | $IC_{50}$ (µM) |
|---|---|---|
| 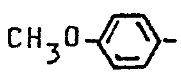 | H | 2,5 |
| 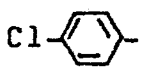 | H | 6 |
| 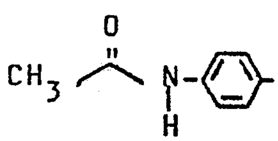 | H | 0,7 |
| 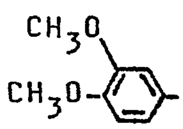 | H | 2 |
| 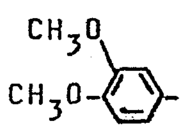 | 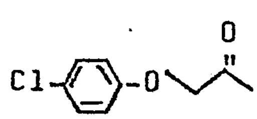 | ⟩ 100 |

Tabelle 6

Hemmung der Thrombozytenaggregation in vitro (nach BORN) durch 10 $\mu M$ ADP induziert

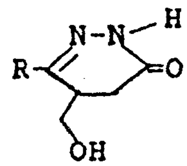

| R | $IC_{50}$ ($\mu M$) |
|---|---|
| 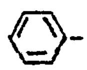 | >100 |
| 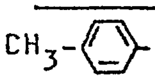 | 150 |
| 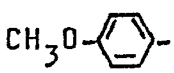 | 50 |
| 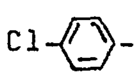 | 70 |
| 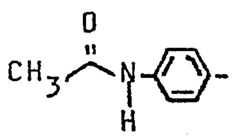 | 10 |
| 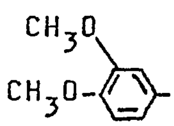 | 9 |
| 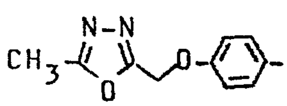 | 20 |
| 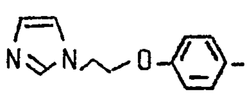 | 150 |
| 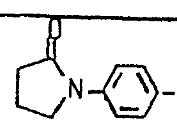 | 20 |

0129791
Ref. 3275/3302
Dr.Va/St

**Tabelle 6**

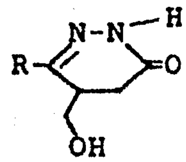

| R | $IC_{50}$ ($\mu M$) |
|---|---|
| 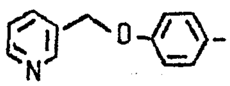 | 36 |
| 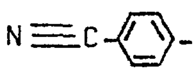 | 20 |

0129791
Ref. 3302
Dr.Va/St

## Patentansprüche

1. Tetrahydropyridazinone der Formel I

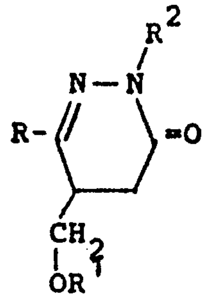

(I)

worin

R

einen der Reste der Formeln

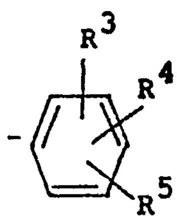

oder

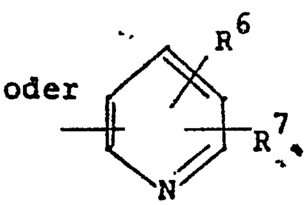

oder

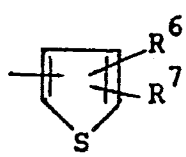

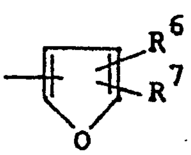

,

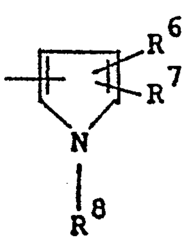

,

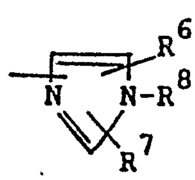

,

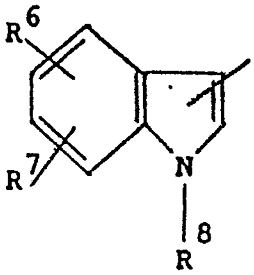

,

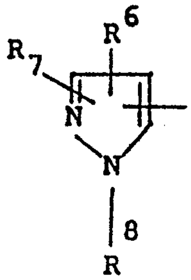

$R^1$ Wasserstoff; Alkyl; Aralkyl, das im Arylteil gegebenenfalls substituiert ist; oder Acyl der Formel $-CO-R^9$;

$R^2$ Wasserstoff; Alkyl, das gegebenenfalls durch basische Gruppen substituiert sein kann; oder gegebenenfalls substituiertes Phenyl;

$R^3$, $R^4$ und $R^5$ unabhängig voneinander Halogen; Alkyl mit 1 bis 5 C-Atomen; -OH; Alkoxy mit 1 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1-C_5)$-oxycarbonyl, eine Gruppe der Formel $-NR^{10}R^{11}$ bedeuten, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

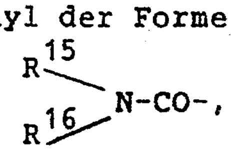

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3

Heteroatomen substituiert sein kann;
$R^4$ und $R^5$ zusätzlich auch Wasserstoff;
$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;
$R^8$ Wasserstoff, Alkyl mit 1 bis 5 C-Atomen, Alkanoyl mit 1 bis 5 C-Atomen oder ein Rest der Formel

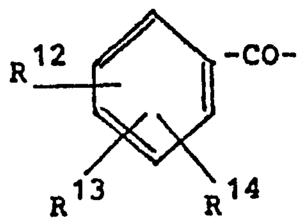

$R^9$ Alkyl mit 1 bis 5 C-Atomen, gegebenenfalls substituiert durch einen gegebenenfalls substituierten Phenoxyrest,

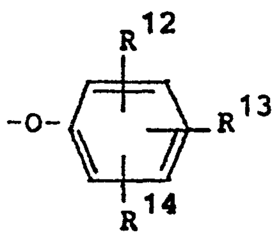

eine Gruppe der Formel

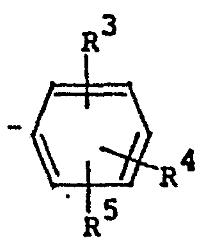

oder Pyridyl;
$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

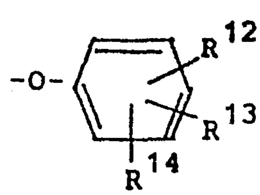

substituiert sein kann; Benzoyl der Formel

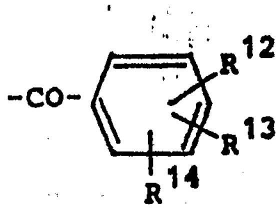

Heteroaryl der Formel

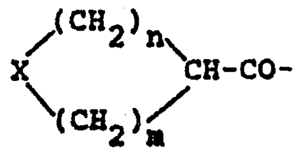

worin n = 2; 3 oder 4,
m = 0; 1 oder 2 ist, wobei vorzugsweise n+m = 3 oder 4 ist,
X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen, Chlor, Brom oder doppeltgebundenen Sauerstoff (=O) substituiert sein kann,

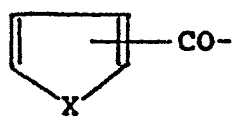 oder 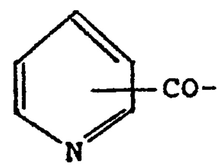 , und

$R^{11}$ Wasserstoff, Alkyl mit 1 bis 5 C-Atomen, Alkanoyl mit 1 bis 5 C-Atomen bedeuten
oder $R^{10}$ und $R^{11}$ gemeinsam mit dem Stickstoffatom, an das sie gebunden sind, einen Pyrrolidin-, Piperidin-, Piperazin-, Morpholin- oder Thiomorpholinring oder einen Ring der Formeln

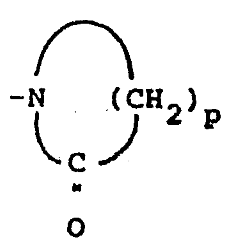

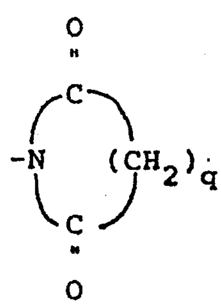

worin p = 3, 4 oder 5; q = 2 oder 3 ist;

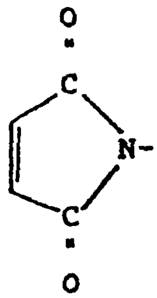 ; 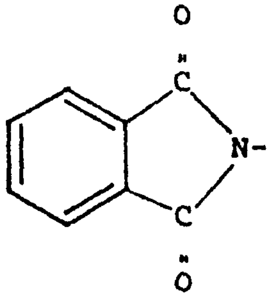 ; 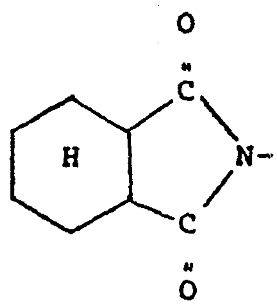 ;

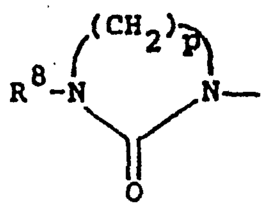 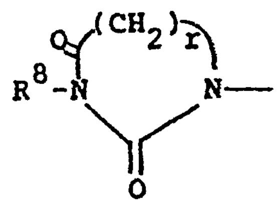

worin r = 1 oder 2 ist,

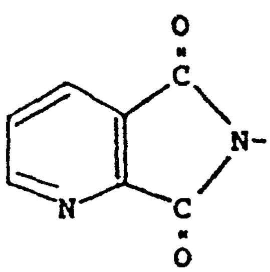 ; 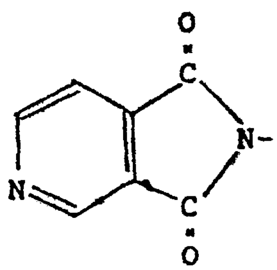 ; 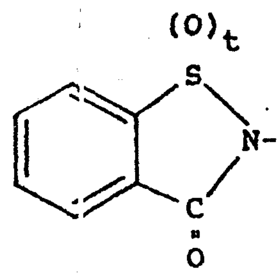

oder 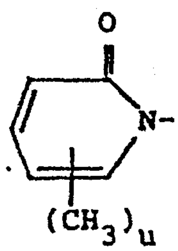

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen,

Alkanoylamino mit 1 bis 5 C-Atomen oder Benzoylamino bedeuten sowie ihre Säureadditionsverbindungen, soweit diese herstellbar sind.

2. Tetrahydropyridazinone der Formel I

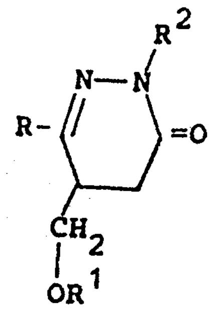

(I)

worin

R einen der Reste der Formeln

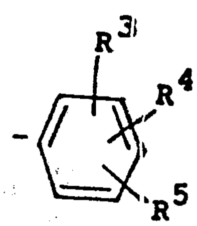

oder

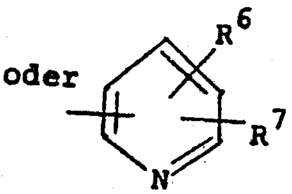

oder

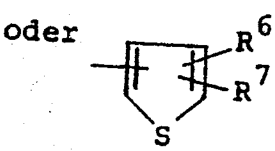

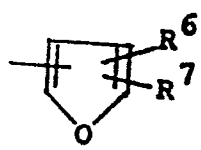

,

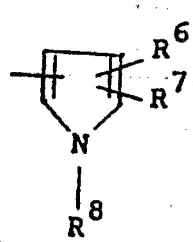

,

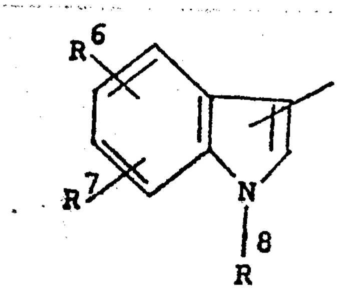

$R^1$ Wasserstoff; Alkyl; Aralkyl, das im Arylteil gegebenenfalls substituiert ist; oder Acyl der Formel $-CO-R^9$;

$R^2$ Wasserstoff; Alkyl, das gegebenenfalls durch basische Gruppen substituiert sein kann; Phenyl oder Phenyl, das durch gegebenenfalls basisch substituierte Alkylgruppen

substituiert ist, $R^3$, $R^4$ und $R^5$ unabhängig voneinander Halogen; Alkyl mit 1 bis 5 C-Atomen; -OH; Alkoxy mit 1 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1$-$C_5)$-oxycarbonyl, eine Gruppe der Formel $-NR^{10}R^{11}$ bedeuten, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

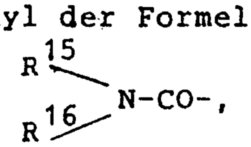

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Halogen, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3 Heteroatomen substituiert sein kann;

$R^4$ und $R^5$ zusätzlich auch Wasserstoff;

$R^6$, $R^7$ und $R^8$ Wasserstoff;

$R^9$ Alkyl mit 1 bis 5 C-Atomen, gegebenenfalls substituiert durch einen gegebenenfalls durch Halogen, Alkyl mit 1 - 4 C-Atomen oder Alkoxy mit 1 - 4 C-Atomen substituierten Phenoxyrest, oder Pyridyl;
$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen substituiert sein kann,oder Benzoyl und $R^{11}$ Wasserstoff bedeuten oder
$R^{10}$ und $R^{11}$ gemeinsam mit dem Stickstoffatom, an das sie gebunden sind, einen Pyrrolidin-, Piperidin-, Piperazin-, Morpholin- oder Thiomorpholinring oder einen Ring der Formeln

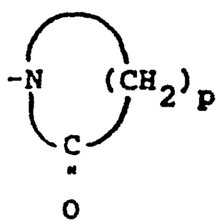
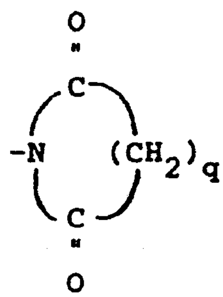

worin p = 3, 4 oder 5; q = 2 oder 3 ist;

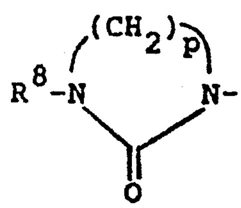
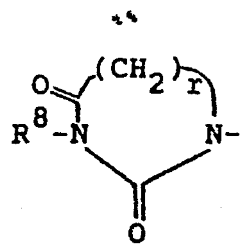

worin r = 1 oder 2 ist, bilden sowie ihre Säureadditionsverbindungen, soweit diese herstellbar sind.

0129791
Ref. 3302
Dr.Va/St

3. Tetrahydropyridazinone der Formel I

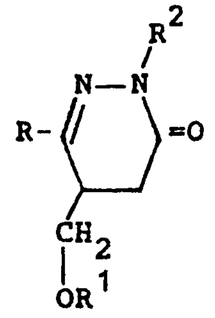

(I)

worin R einen der Reste der Formeln

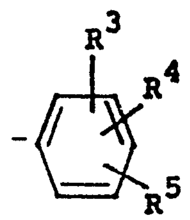 oder 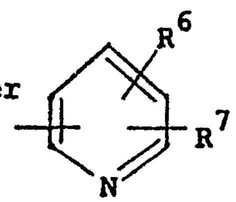 oder 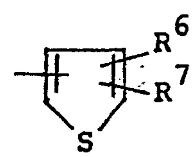

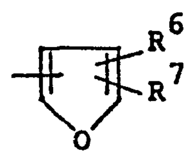 , 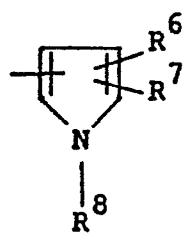 , 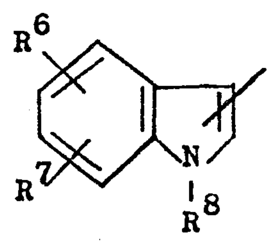

$R^1$ Wasserstoff oder Acyl der Formel $-CO-R^9$;
$R^2$ Wasserstoff, Alkyl, Phenyl oder Phenyl, das durch gegebenenfalls basisch substituierte Alkylgruppen substituiert ist;
$R^3$, $R^4$ und $R^5$ unabhängig voneinander Halogen; Alkyl mit 1 bis 5 C-Atomen; -OH; Alkoxy mit 1 bis 5 C-Atomen; Alkanoyloxy mit 1 bis 5 C-Atomen; Alkylmercapto mit 1 bis 5 C-Atomen; $-NH_2$; Dialkylamino mit insgesamt 2 bis 5 C-Atomen; Alkanoylamino mit 1 bis 4 C-Atomen; und $R^3$ und $R^4$ außerdem Nitro; Cyan; Alkylmercapto; eine Gruppe der Formel $-NR^{10}R^{11}$ bedeuten, wobei für $R^3$ und $R^4$ stehendes Alkyl und Alkoxy in dem Alkylrest noch durch Alkoxy mit 1 bis 5, vorzugsweise 1 oder 2, C-Atomen, Aminocarbonyl der Formel

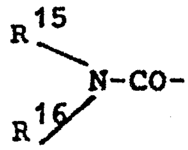

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Chlor und Brom substituiert sein kann, bedeuten oder durch einen 5- oder 6-gliedrigen Heterocyclus mit 1 bis 3 Heteroatomen substituiert sein kann;

$R^4$ und $R^5$ zusätzlich auch Wasserstoff;

$R^6$, $R^7$ und $R^8$ Wasserstoff;

$R^9$ Alkyl mit 1 bis 5 C-Atomen, gegebenenfalls substituiert durch einen gegebenenfalls durch Halogen, Alkyl mit 1 - 4 C-Atomen oder Alkoxy mit 1 - 4 C-Atomen substituierten Phenoxyrest, oder Pyridyl;

$R^{10}$ Alkanoyl mit 1 bis 5 C-Atomen oder Benzoyl und

$R^{11}$ Wasserstoff bedeuten oder

$R^{10}$ und $R^{11}$ gemeinsam mit dem Stickstoffatom, an das sie gebunden sind, einen Ring der Formeln

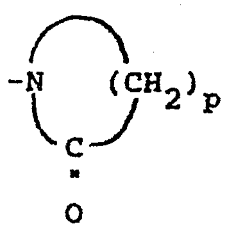 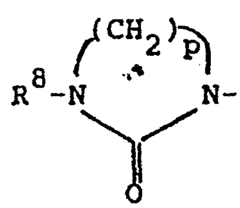 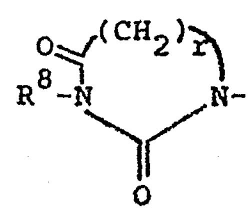

worin p = 3, 4 oder 5; und r = 1 oder 2 ist, bilden sowie ihre Säureadditionsverbindungen, soweit diese herstellbar sind.

0129791
Ref.3275/3302
Dr.Va/St

4. Tetrahydropyridazinone gemäß den Ansprüchen 1 bis 3, dadurch gekennzeichnet, daß $R^1$ für Wasserstoff; Alkyl mit 1 oder 2 C-Atomen; Alkanoyl mit 1 bis 3 C-Atomen; Benzoyl oder Nicotinoyl steht.

5. Tetrahydropyridazinone gemäß den Ansprüchen 1 bis 3, dadurch gekennzeichnet, daß $R^1$ und $R^2$ Wasserstoff bedeuten.

6. 6-(3,4-Dimethoxyphenyl)-5-hydroxymethyl-2,3,4,5-tetrahydro-pyridazin-3-on und seine Säureadditionsverbindungen.

7. Verfahren zur Herstellung der Tetrahydropyridazinone des Anspruchs 1, dadurch gekennzeichnet, daß man ein Acyl- -butyrolacton der Formel II mit Hydrazin oder Hydrazinderivaten der Formel III nach dem Schema

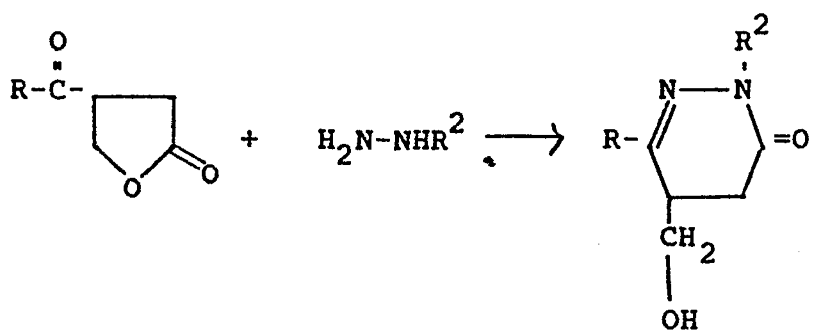

(II) (III) (Ia)

worin R und $R^2$ die obengenannten Bedeutungen haben in an sich bekannter Weise umsetzt und, sofern erfindungsgemäße Verbindungen hergestellt werden sollen, in denen $R^1$ von Wasserstoff verschieden ist, und anschließend die 5-ständige Hydroxymethylgruppe mit einem Veretherungs- bzw. Veresterungsmittel der Formel IV

$$R^1 - X \qquad (IV),$$

worin $R^1$ die obengenannte Bedeutung hat und X ein als Anion abspaltbarer Rest ist, verethert bzw. verestert.

8. Verfahren gemäß Anspruch 7 zur Herstellung von Tetrahydropyridazinonen des Anspruchs 1, in denen $R^1$ und $R^2$ Wasserstoff bedeuten, dadurch gekennzeichnet, daß man ein Acyl-γ-butyrolacton der in Anspruch 4 angegebenen Formel II mit Hydrazin oder Hydrazinhydrat (Reaktant: $NH_2-NH_2$) in an sich bekannter Weise umsetzt.

9. Verfahren zur Herstellung von Tetrahydropyridazinonen des Anspruchs 1, in denen $R^1$ und/oder $R^2$ von Wasserstoff verschieden sind, dadurch gekennzeichnet, daß man ein Tetrahydropyridazinon des Anspruchs 1, in dem $R^1$ und $R^2$ Wasserstoff sind, nacheinander in beliebiger Abfolge:
a) zur Veretherung bzw. Veresterung der 5-ständigen Hydroxymethylgruppe mit einem Veretherungs-bzw. Veresterungsmittel der Formel IV

$$R^1 - X \qquad (IV)$$

worin $R^1$ die obengenannte Bedeutung hat und X ein als Anion abspaltbarer Rest ist, in an sich bekannter Weise umsetzt und gegebenenfalls,
b) zur Alkylierung bzw. Arylierung der 2-Stellung des Tetrahydropyridazinons mit einem Alkylierungs- bzw. Arylierungsmittel der Formel V

$$R^2 - X \qquad (V)$$

umsetzt.

10. Verwendung von Tetrahydropyridazinonen der Formel I

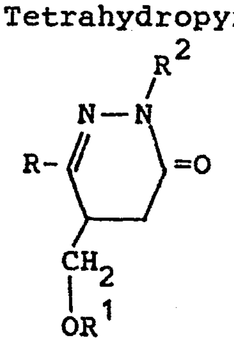

(I)

worin R, $R^1$ und $R^2$ die im Anspruch 1 angegebenen Bedeutungen haben und R zusätzlich einen unsubstituierten Phenylrest ($R^3$ = $R^4$ = $R^5$ = Wasserstoff) bedeutet, sowie

ihrer Säureadditionsverbindungen zur Behandlung, Bekämpfung und Vorbeugung von Erkrankungen des Herzens und des Kreislaufsystems einschließlich thromboembolischer Erkrankungen.

11. Arzneimittelzubereitung enthaltend, neben den üblichen in der Galenik eingesetzten Hilfs- und Trägerstoffen, eine wirksame Dosis eines Tetrahydropyridazinons der Formel I,

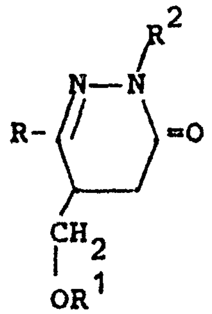

(I)

worin
R, $R^1$ und $R^2$ die im Anspruch 1 angegebenen Bedeutungen haben und R zusätzlich einen unsubstituierten Phenylrest ($R^3 = R^4 = R^5 =$ Wasserstoff) bedeutet, oder eine Säureadditionsverbindung davon.

12. Acyl-γ-butyrolactone der Formel II, worin
R einen der Reste der Formeln

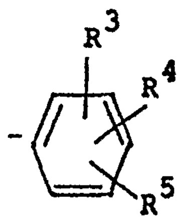
oder
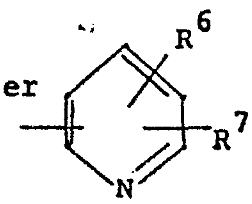
oder
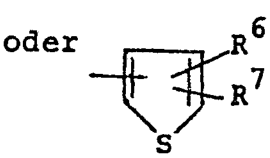

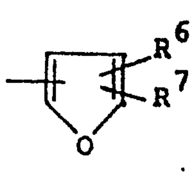
,
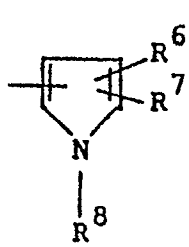
,
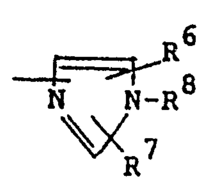
,

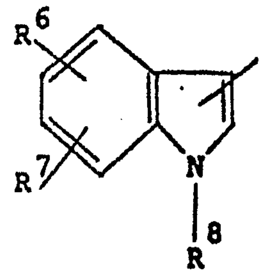 , 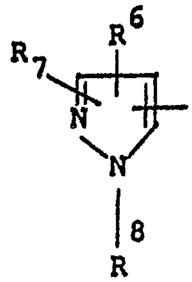

$R^3$, $R^4$ und $R^5$ unabhängig voneinander Fluor, Chlor, Jod, o-Brom oder m-Brom; Alkyl mit 2 bis 5 C-Atomen, o- oder m-Methyl; -OH; Alkoxy mit 2 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^4$ und $R^5$ zusätzlich auch Wasserstoff; einer der Reste $R^3$, $R^4$ oder $R^5$ Methoxy, die beiden anderen Wasserstoff; zwei der Reste $R^3$, $R^4$ oder $R^5$ 2,3-, 2,4-, 2,5-, 2,6-, 3,5-Dimethoxy und der dritte Wasserstoff, oder alle drei der Reste $R^3$, $R^4$ und $R^5$ Methoxy;
und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1$-$C_5)$-oxycarbonyl, eine Gruppe der Formel $-NR^{10}R^{11}$, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

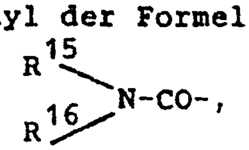 ,

wobei $R^{15}$ und $R^{16}$ unabhängig voneinander Wasserstoff, Alkyl mit 1 bis 5 C-Atomen, das seinerseits durch Alkoxy

mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3 Heteroatomen substituiert sein kann;

$R^4$ und $R^5$ zusätzlich auch Wasserstoff;

$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;

$R^8$ Wasserstoff, Alkyl mit 1 bis 5 C-Atomen, Alkanoyl mit 1 bis 5 C-Atomen oder ein Rest der Formel

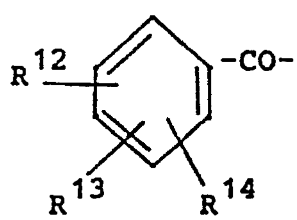

$R^9$ Alkyl mit 1 bis 5 C-Atomen, gegebenenfalls substituiert durch einen gegebenenfalls substituierten Phenoxyrest,

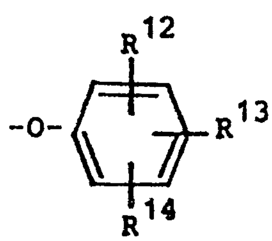

eine Gruppe der Formel

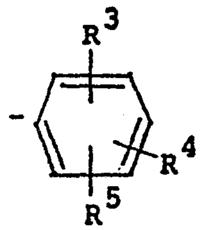

oder Pyridyl;

$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

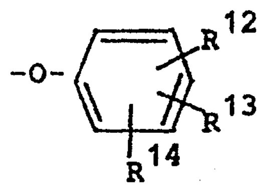

substituiert sein kann; Benzoyl der Formel

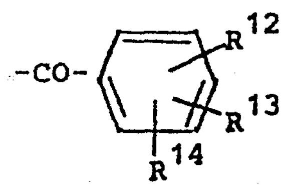

Heteroaryl der Formel

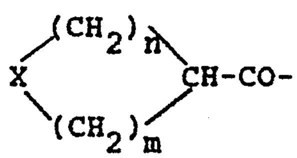

worin n = 2; 3 oder 4,
m = 0; 1 oder 2 ist, wobei vorzugsweise n+m = 3 oder 4 ist,
X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen, Chlor, Brom oder doppeltgebundenen Sauerstoff (=O) substituiert sein kann,

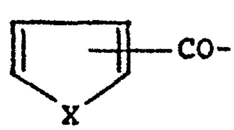 oder 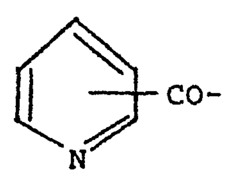 , und

$R^{11}$ Wasserstoff, Alkyl mit 1 bis 5 C-Atomen, Alkanoyl mit 1 bis 5 C-Atomen bedeuten
oder $R^{10}$ und $R^{11}$ gemeinsam mit dem Stickstoffatom, an das

sie gebunden sind, einen Pyrrolidin-, Pyrimidin-, Piperidin-, Piperazin-, Morpholin- oder Thiomorpholinring oder einen Ring der Formeln

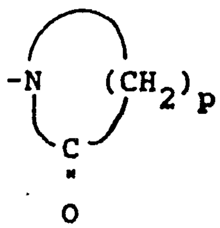

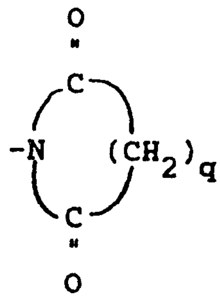

worin p = 3, 4 oder 5; q = 2 oder 3;

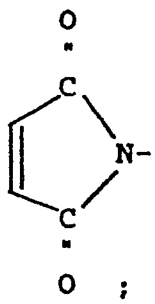

;

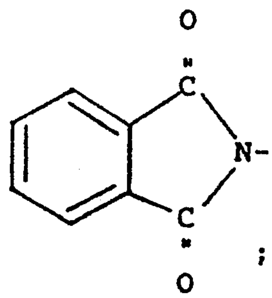

;

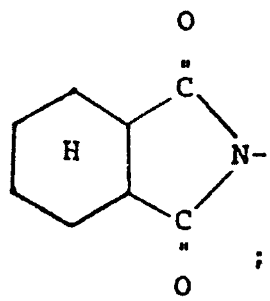

;

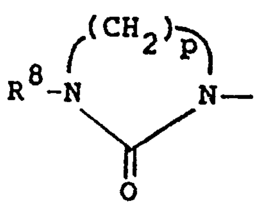

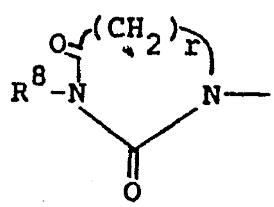

worin r = 1 oder 2 ist,

0129791
Ref. 3302
Dr.Va/St

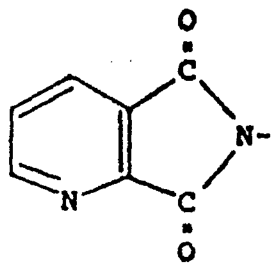 ; 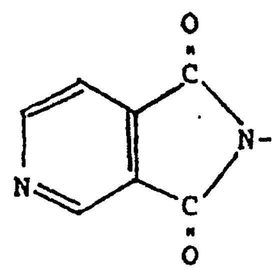 ; 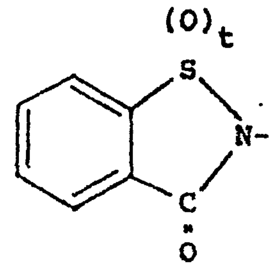

oder 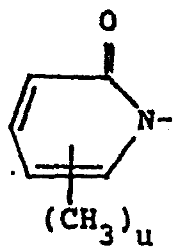

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen, Alkanoylamino mit 1 bis 5 C-Atomen oder Benzoylamino bedeuten.

13. Verfahren zur Herstellung von Acyl-γ-butyrolactonen der Formel II, dadurch gekennzeichnet, daß man eine in 4-Stellung substituierte 4-Ketobuttersäure der Formel

$$R - \overset{\overset{O}{\|}}{C}-CH_2CH_2-COOH \qquad \text{(VI)}$$

mit Formaldehyd bei 20 bis 100°C, in Gegenwart eines basischen Katalysators umsetzt, anschließend das Reaktionsgemisch ansäuert und bis zum Abschluß der Ringschlußreaktion bei 20 bis 120°C hält.

0129791
Ref. 3302
Dr.Va/St

Patentansprüche für den Vertragsstaat Österreich

1. Verfahren zur Herstellung von Tetrahydropyridazinonen der Formel I

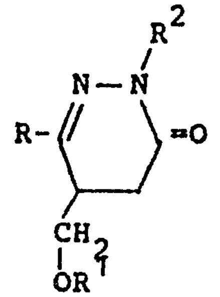

(I)

worin

R

einen der Reste der Formeln

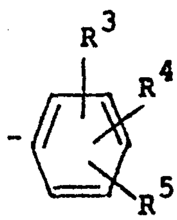

oder

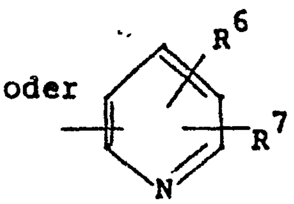

,

oder

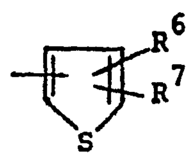

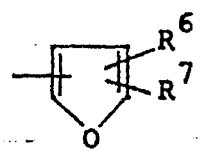

,

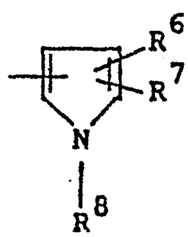

,

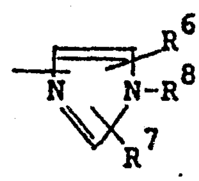

,

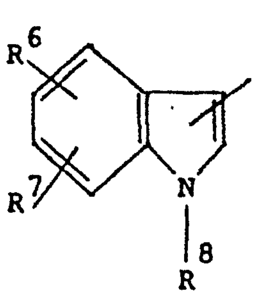

,

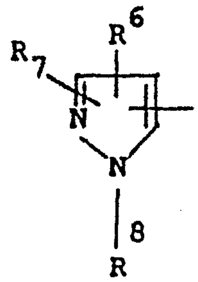

$R^1$ **Wasserstoff**; Alkyl; Aralkyl, das im Arylteil gegebenenfalls substituiert ist; oder Acyl der Formel $-CO-R^9$;

$R^2$ Wasserstoff; Alkyl, das gegebenenfalls durch basische Gruppen substituiert sein kann; oder gegebenenfalls substituiertes Phenyl;

$R^3$, $R^4$ und $R^5$ unabhängig voneinander Halogen; Alkyl mit 1 bis 5 C-Atomen; -OH; Alkoxy mit 1 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1-C_5)$-oxycarbonyl, eine Gruppe der Formel $-NR^{10}R^{11}$ bedeuten, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

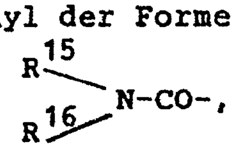

wobei $R^{15}$ und $R^{16}$ unabhängig voneinander Wasserstoff, Alkyl mit 1 bis 5 C-Atomen, das seinerseits durch Alkoxy mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3

Heteroatomen substituiert sein kann;
$R^4$ und $R^5$ zusätzlich auch Wasserstoff;
$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;
$R^8$ Wasserstoff, Alkyl mit 1 bis 5 C-Atomen, Alkanoyl mit 1 bis 5 C-Atomen oder ein Rest der Formel

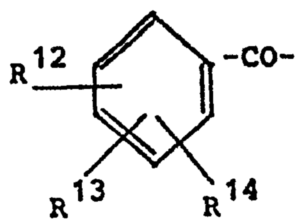

$R^9$ Alkyl mit 1 bis 5 C-Atomen, gegebenenfalls substituiert durch einen gegebenenfalls substituierten Phenoxyrest,

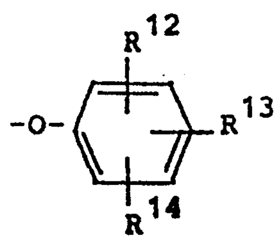

eine Gruppe der Formel

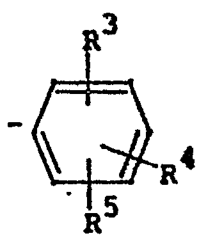

oder Pyridyl;
$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

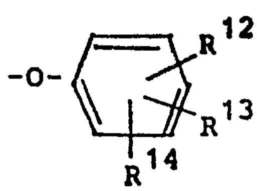

substituiert sein kann; Benzoyl der Formel

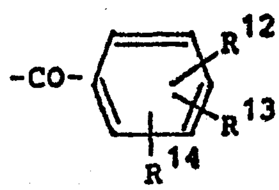

Heteroaryl der Formel

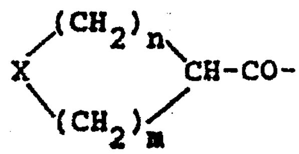

worin n = 2; 3 oder 4, m = 0; 1 oder 2 ist, wobei vorzugsweise n+m = 3 oder 4 ist,
X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen, Chlor, Brom oder doppeltgebundenen Sauerstoff (=O) substituiert sein kann,

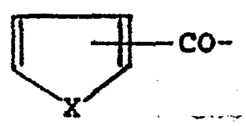 oder 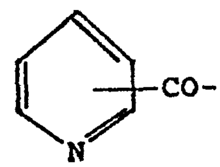 , und

$R^{11}$ Wasserstoff, Alkyl mit 1 bis 5 C-Atomen, Alkanoyl mit 1 bis 5 C-Atomen bedeuten
oder $R^{10}$ und $R^{11}$ gemeinsam mit dem Stickstoffatom, an das sie gebunden sind, einen Pyrrolidin-, Piperidin-, Piperazin-, Morpholin- oder Thiomorpholinring oder einen Ring der Formeln

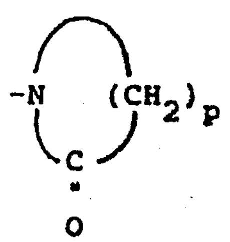 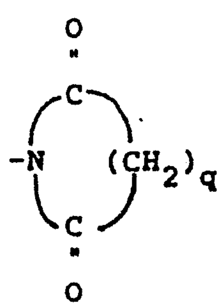

worin p = 3, 4 oder 5; q = 2 oder 3 ist;

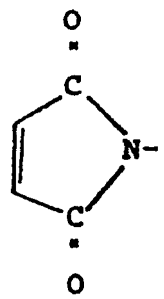 ; 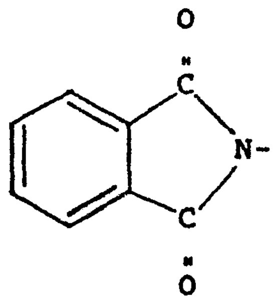 ; 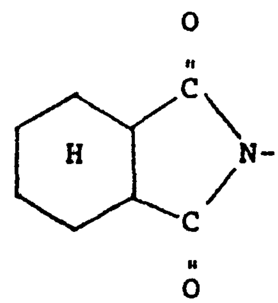 ;

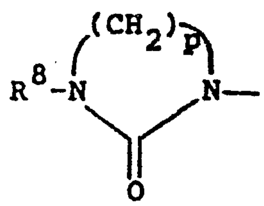 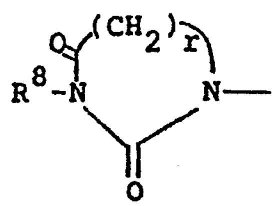

worin r = 1 oder 2 ist,

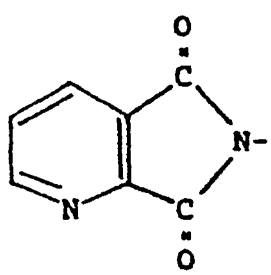 ; 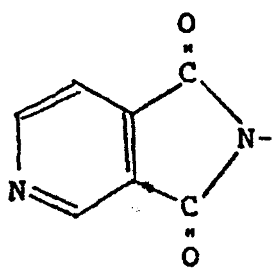 ; 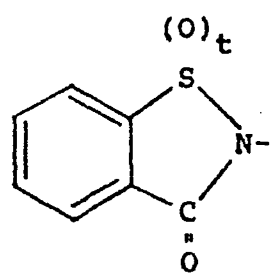

oder 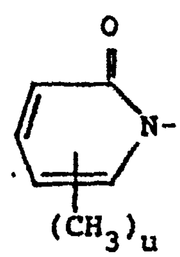

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen,

Alkanoylamino mit 1 bis 5 C-Atomen oder Benzoylamino bedeuten sowie ihre Säureadditionsverbindungen, soweit diese herstellbar sind, dadurch gekennzeichnet, daß man ein Acyl-γ-butyrolacton der Formel II mit Hydrazin oder Hydrazinderivaten der Formel III nach dem Schema

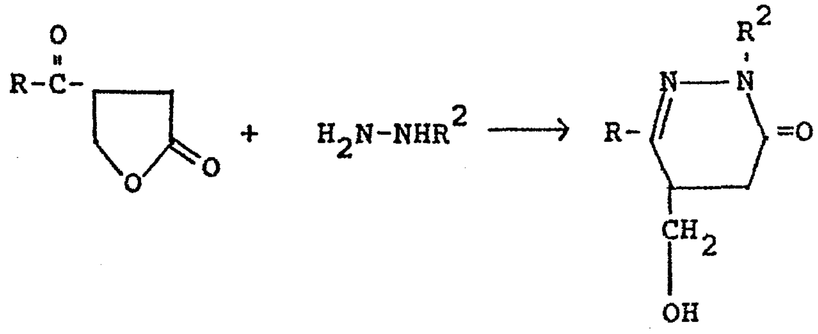

(II) (III) (Ia)

worin R und $R^2$ die obengenannten Bedeutungen haben, in an sich bekannter Weise umsetzt und, sofern erfindungsgemäße Verbindungen hergestellt werden sollen, in denen $R^1$ von Wasserstoff verschieden ist, anschließend die 5-ständige Hydroxymethylgruppe mit einem Veretherungs-bzw. Veresterungsmittel der Formel IV

$$R^1 - X \qquad (IV),$$

worin $R^1$ die obengenannte Bedeutung hat und X ein als Anion abspaltbarer Rest ist, verethert bzw. verestert und das erhaltene Produkt anschließend gewünschtenfalls mit einer Säure zur Säureadditionsverbindung umsetzt.

2. Verfahren gemäß Anspruch 1 zur Herstellung von Tetrahydropyridazinonen der Formel I, worin R die im Anspruch 1 genannte Bedeutung hat und $R^1$ und $R^2$ Wasserstoff bedeuten, dadurch gekennzeichnet, daß man ein Acyl-γ-butyrolacton der in Anspruch 1 angegebenen Formel II mit Hydrazin oder Hydrazinhydrat (Reaktant: $NH_2$-$NH_2$) in an sich bekannter Weise umsetzt.

0129791
Ref.3275/3302
Dr.Va/St

3. Verfahren zur Herstellung von Tetrahydropyridazinonen der Formel I, worin R die im Anspruch 1 genannte Bedeutung hat und in denen $R^1$ und/oder $R^2$ von Wasserstoff verschieden sind, dadurch gekennzeichnet, daß man ein Tetrahydropyridazinon der Formel I, in dem $R^1$ und $R^2$ Wasserstoff sind, nacheinander in beliebiger Abfolge:
a) zur Veretherung bzw. Veresterung der 5-ständigen Hydroxymethylgruppe mit einem Veretherungs-bzw. Veresterungsmittel der Formel IV

$$R^1 - X \qquad (IV)$$

worin $R^1$ die obengenannte Bedeutung hat und X ein als Anion abspaltbarer Rest ist, in an sich bekannter Weise umsetzt und gegebenenfalls,
b) zur Alkylierung bzw. Arylierung der 2-Stellung des Tetrahydropyridazinons mit einem Alkylierungs- bzw. Arylierungsmittel der Formel V

$$R^2 - X \qquad (V)$$

umsetzt.

4. Verwendung von Tetrahydropyridazinonen der Formel I

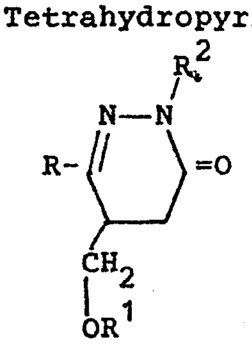

(I)

worin R, $R^1$ und $R^2$ die im Anspruch 1 angegebenen Bedeutungen haben und R zusätzlich einen unsubstituierten Phenylrest ($R^3$ = $R^4$ = $R^5$ = Wasserstoff) bedeutet, sowie ihrer Säureadditionsverbindungen zur Behandlung, Bekämpfung und Vorbeugung von Erkrankungen des Herzens und des Kreislaufsystems einschließlich thromboembolischer Erkrankungen.

5. Arzneimittelzubereitung enthaltend, neben den üblichen in der Galenik eingesetzten Hilfs- und Trägerstoffen,

0129791
Ref.3275/3302
Dr.Va/St

eine wirksame Dosis eines Tetrahydropyridazinons der Formel I,

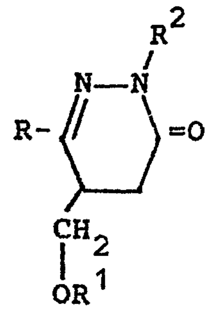

(I)

worin

R, $R^1$ und $R^2$ die im Anspruch 1 angegebenen Bedeutungen haben und R zusätzlich einen unsubstituierten Phenylrest ($R^3$ = $R^4$ = $R^5$ = Wasserstoff) bedeutet, oder eine Säureadditionsverbindung davon.

6. Verfahren zur Herstellung von Acyl-γ-butyrolactonen der Formel II

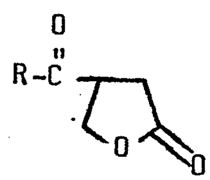

worin R einen der Reste der Formeln

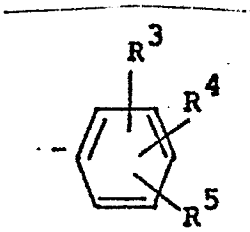

oder

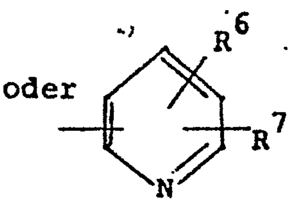

oder

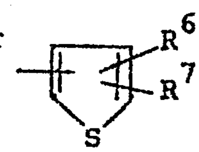

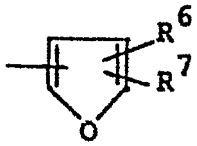

,

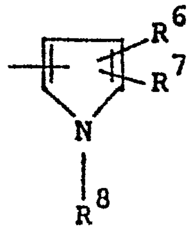

,

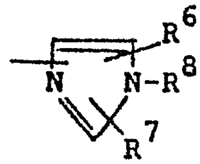

,

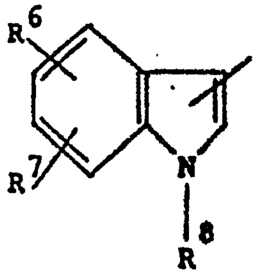

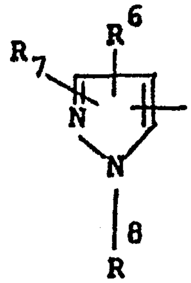

$R^3$, $R^4$ und $R^5$ unabhängig voneinander Fluor, Chlor, Jod, o-Brom oder m-Brom; Alkyl mit 2 bis 5 C-Atomen, o- oder m-Methyl; -OH; Alkoxy mit 2 bis 5 C-Atomen; Alkanoyloxy mit 1 bis 5 C-Atomen; Benzoyloxy; Alkylmercapto mit 1 bis 5 C-Atomen; $-NH_2$; Monoalkylamino mit 1 bis 5 C-Atomen; Dialkylamino mit insgesamt 2 bis 5 C-Atomen; Alkanoylamino mit 1 bis 4 C-Atomen; und $R^4$ und $R^5$ zusätzlich auch Wasserstoff; einer der Reste $R^3$, $R^4$ oder $R^5$ Methoxy, die beiden anderen Wasserstoff; zwei der Reste $R^3$, $R^4$ oder $R^5$ 2,3-, 2,4-, 2,5-, 2,6-, 3,5-Dimethoxy und der dritte Wasserstoff, oder alle drei der Reste $R^3$, $R^4$ und $R^5$ Methoxy;
und $R^3$ und $R^4$ außerdem Nitro; Cyan; Alkylmercapto, Alkylsulfoxy und Alkylsulfonyl mit jeweils 1 bis 5 C-Atomen, Alkyl-$(C_1-C_5)$-oxycarbonyl, eine Gruppe der Formel $-NR^{10}R^{11}$, wobei für $R^3$ und $R^4$ stehendes Alkyl, Alkoxy, Alkylamino, Dialkylamino, Alkylmercapto, Alkylsulfoxy und Alkylsulfonyl in dem Alkylteil noch durch Hydroxy, Alkoxy mit 1 bis 5, vorzugsweise 1 oder 2, C-Atomen, Carboxyl, Alkoxycarbonyl mit insgesamt 2 bis 7 C-Atomen, Aminocarbonyl der Formel

$$\begin{matrix} R^{15} \\ R^{16} \end{matrix} \Big> N-CO- ,$$

wobei $R^{15}$ und $R^{16}$ unabhängig voneinander Wasserstoff, Alkyl mit 1 bis 5 C-Atomen, das seinerseits durch Alkoxy

mit 1 bis 4 C-Atomen, Halogen, insbesondere Chlor und Brom, $-NH_2$, Mono- und Dialkylamino mit 1 bis 4 C-Atomen in den Alkylgruppen substituiert sein kann, bedeuten oder wobei $R^{15}$ und $R^{16}$ zusammen mit dem N-Atom, an das sie gebunden sind, den Rest eines 5- oder 6-gliedrigen, gegebenenfalls ein weiteres Heteroatom aufweisenden Heterocyclus, wie z.B. Pyrrolidin, Piperidin, Piperazin, Alkylpiperazin, Morpholin, Thiomorpholin bilden, Amino, Monoalkylamino mit 1 bis 5, vorzugsweise 1 oder 2, C-Atomen, Dialkylamino mit insgesamt 2 bis 6 C-Atomen oder durch einen 5- oder 6-gliedrigen Heterocyclus mit 1 bis 3 Heteroatomen substituiert sein kann;

$R^4$ und $R^5$ zusätzlich auch Wasserstoff;

$R^6$ und $R^7$ unabhängig voneinander Wasserstoff; Halogen, insbesondere Chlor und Brom; Amino, Monoalkylamino mit 1 bis 5 C-Atomen, Dialkylamino mit insgesamt 2 bis 6 C-Atomen, Hydroxy, Alkoxy mit 1 bis 5 C-Atomen oder Alkyl mit 1 bis 5 C-Atomen;

$R^8$ Wasserstoff, Alkyl mit 1 bis 5 C-Atomen, Alkanoyl mit 1 bis 5 C-Atomen oder ein Rest der Formel

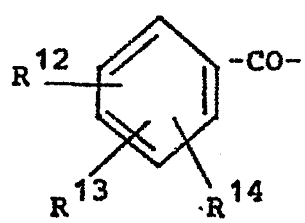

$R^9$ Alkyl mit 1 bis 5 C-Atomen, gegebenenfalls substituiert durch einen gegebenenfalls substituierten Phenoxyrest,

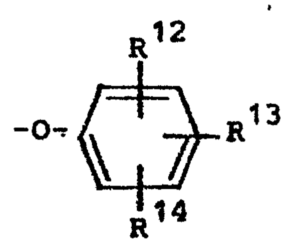

eine Gruppe der Formel

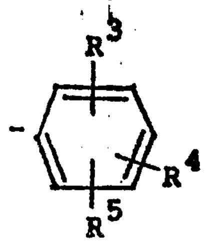

oder Pyridyl;

$R^{10}$ Alkanoyl mit 1 bis 5 C-Atomen, das gegebenenfalls durch -OH, $-NH_2$, Halogen, insbesondere Chlor oder Brom, Alkoxy mit 1 bis 5 C-Atomen oder Phenoxy der Formel

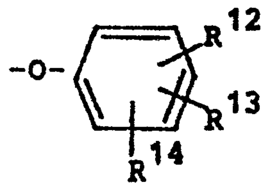

substituiert sein kann; Benzoyl der Formel

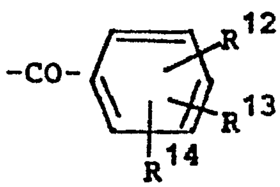

Heteroaryl der Formel

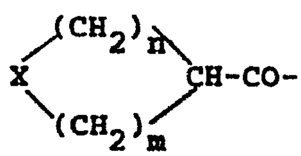

worin n = 2; 3 oder 4,

m = 0; 1 oder 2 ist, wobei vorzugsweise n+m = 3 oder 4 ist,

X für Sauerstoff, Schwefel oder eine Gruppe der Formel $=N-R^8$ steht, wobei der heterocyclische Kern noch durch Alkyl mit 1 bis 5, vorzugsweise 1 oder 2, C-Atomen, Chlor, Brom oder doppeltgebundenen Sauerstoff (=O) substituiert sein kann,

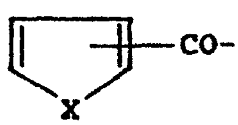 oder 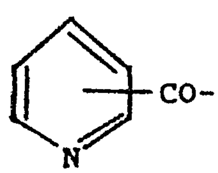 , und

$R^{11}$ Wasserstoff, Alkyl mit 1 bis 5 C-Atomen, Alkanoyl mit 1 bis 5 C-Atomen bedeuten

oder $R^{10}$ und $R^{11}$ gemeinsam mit dem Stickstoffatom, an das

sie gebunden sind, einen Pyrrolidin-, Pyrimidin-, Piperidin-, Piperazin-, Pyrimidin-, Morpholin- oder Thiomorpholinring oder einen Ring der Formeln

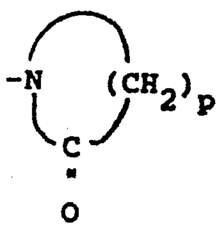

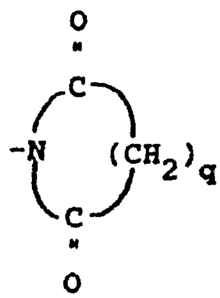

worin p = 3, 4 oder 5; q = 2 oder 3;

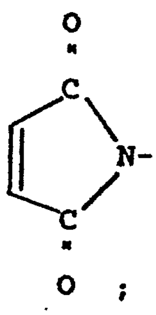 ; 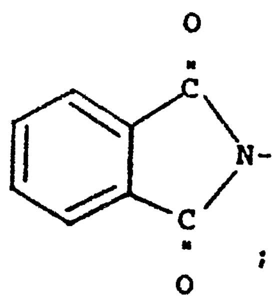 ; 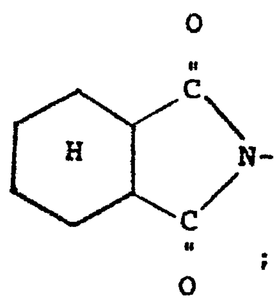 ;

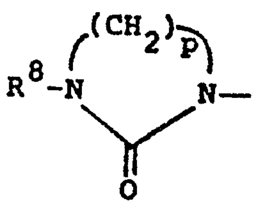

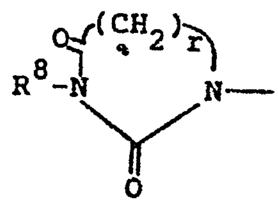

worin r = 1 oder 2 ist,

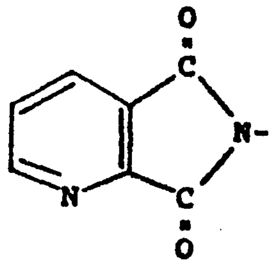 ; 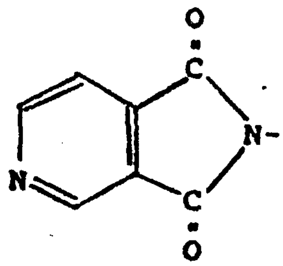 ; 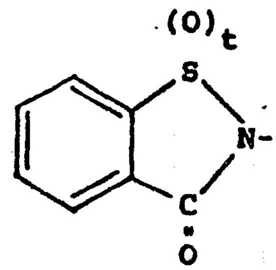

oder 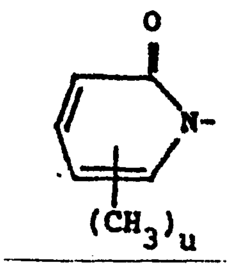

wobei u = 0, 1 oder 2 und t = 0, 1 oder 2 ist, der gegebenenfalls noch durch Alkyl mit 1 bis 5, vorzugsweise 1 bis 2, C-Atomen substituiert sein kann, bilden, und $R^{12}$ bis $R^{14}$ Wasserstoff, Alkyl mit 1 bis 5 C-Atomen, Alkoxy mit 1 bis 5 C-Atomen, Nitro, Cyan, Halogen, insbesondere Chlor oder Brom, Amino, Monoalkylamino mit 1 bis 5 C-Atomen, Dialkylamino mit insgesamt 2 bis 6 C-Atomen, Alkanoylamino mit 1 bis 5 C-Atomen oder Benzoylamino bedeuten, dadurch gekennzeichnet, daß man eine in 4-Stellung substituierte 4-Ketobuttersäure der Formel

$$R - \overset{O}{\overset{\|}{C}}-CH_2CH_2-COOH \qquad (VI)$$

worin R die oben genannte Bedeutung hat, mit Formaldehyd bei 20 bis 100°C, in Gegenwart eines basischen Katalysators umsetzt, anschließend das Reaktionsgemisch ansäuert und bis zum Abschluß der Ringschlußreaktion bei 20 bis 120°C hält.